(19) Europäisches Patentamt / European Patent Office / Office européen des brevets

(11) **EP 2 571 086 B1**

(12) **EUROPEAN PATENT SPECIFICATION**

(45) Date of publication and mention
of the grant of the patent:
**24.10.2018 Bulletin 2018/43**

(51) Int Cl.:
**H01M 8/04** *(2016.01)* **G01N 25/18** *(2006.01)*
**G01N 33/22** *(2006.01)* **H01M 8/10** *(2016.01)*

(21) Application number: **12184190.2**

(22) Date of filing: **13.09.2012**

(54) **Electric power generation system and gas measuring system**

Verfahren zur Erzeugung elektrischen Stroms und Gasmesssystem

Système de génération de puissance électrique et système de mesure de gaz

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.09.2011 JP 2011200370**

(43) Date of publication of application:
**20.03.2013 Bulletin 2013/12**

(73) Proprietor: **Azbil Corporation
Chiyoda-ku
Tokyo 100-6419 (JP)**

(72) Inventor: **Ooishi, Yasuharu
Tokyo, 100-6419 (JP)**

(74) Representative: **Lavoix
Bayerstrasse 83
80335 München (DE)**

(56) References cited:
**WO-A1-2004/008136     US-A1- 2008 113 232
US-A1- 2011 185 789     US-B2- 6 713 040
US-B2- 6 997 037**

**Description**

Cross-Reference to Related Application

The present application claims priority to Japanese patent application No. 2011-200370 filed on September 14, 2011.

Technical Field

The present disclosure relates to an electric power generating system and a gas measuring system, relating to a gas inspecting technology.

Related Art

In recent years, fuel cells have attracted attention as household power sources due to their low rate of consumption of primary energy, such as kerosene or natural gas, and their low rate of exhaust of carbon dioxide ($CO_2$). (See, for example, Japanese Unexamined Patent Application Publication H10-284104 and Japanese Unexamined Patent Application Publication 2002-315224). There are various types of fuel cells, where solid-state polymeric fuel cells generate electricity through providing an oxidizing agent to a positive electrode and a reducing agent (the fuel) to a negative electrode, with an ion exchange membrane interposed therebetween. Hydrogen, obtained through reformation of utility gas, is used as the fuel.

As described above, the solid-state polymeric fuel cell generates electricity through a chemical reaction of the hydrogen, provided as the fuel, with oxygen. Because of this, there is the need for a technology able to measure accurately the amount of hydrogen provided to the fuel cell. Moreover, there is the need for a technology able to measure accurately the quantity of atoms that structure molecules included in a gas in a variety of fields, not limited to fuel cells. Given this, one object of the present disclosure is to provide an electric power generating system and gas measuring system able to evaluate the number of atoms that structure molecules included in a gas.

US 2011/185789 A1 concerns a calorific value calculation formula generating system is equipped with a measuring mechanism for measuring heat dissipation constant values or thermal conductivity values of each of plural mixed gases each containing plural kinds of gas components at plural temperatures; and a formula generation module for generating a calorific value calculation formula having heat dissipation constant or thermal conductivities at the plural temperatures as independent variables and a calorific value as a dependent variable on the basis of calorific value values of the plural mixed gases and the heat dissipation constant values or the thermal conductivity values measured at the plural temperatures.

Summary

The present disclosure discloses an electric power generating system includes (a) a temperature measuring element being in contact with a gas, (b) a heating element being in contact with the gas and producing heat at a plurality of heat producing temperatures, (c) a measuring unit measuring a value for an electric signal from a temperature measuring element that is dependent on the temperature the gas, and a value for an electric signal from a heating element at each of a plurality of heat producing temperatures, (d) an equation storage device storing a first equation that includes independent variables that represent electric signals from the temperature measuring element and electric signals from the heating element at the plurality of heat producing temperatures and a dependent variable that represents the sum of the products of the respective numbers of hydrogen atoms that structure the molecules included in the gas, the atomic weight of hydrogen, and the volumetric ratios of the respective molecules, (e) a calculator calculating a value that represents the sum of the products of the respective numbers of hydrogen atoms that structure the molecules included in the gas, the atomic weight of hydrogen, and the volumetric ratios of the respective molecules, by substituting, into the independent variables in the first equation, values for the electric signals from the measuring element and values for the electric signals from the heating element, (f) a fuel cell being supplied with hydrogen extracted from the gas; and (g) a controlling device controlling the amount of hydrogen provided to the fuel cell, based on the value indicated by the calculated product.

The present disclosure discloses a gas measuring system including (a) a temperature measuring element being in contact with a gas, (b) a heating element being in contact with the gas and producing heat at a plurality of heat producing temperatures, (c) a measuring unit measuring a value for an electric signal from a temperature measuring element that is dependent on the temperature the gas, and a value for an electric signal from a heating element at each of a plurality of heat producing temperatures, (d) an equation storage device storing an equation that includes independent variables that represent electric signals from the temperature measuring element and electric signals from the heating element at the plurality of heat producing temperatures and a dependent variable that represents the sum of the products of the respective numbers of atoms that structure the molecules included in the gas, the atomic weights of the atoms, and the volumetric ratios of the respective molecules, and (e) a calculator calculating a value that represents the sum of the products of the respective numbers of atoms that structure the molecules included in the gas, the atomic weights of the atoms, and the volumetric ratios of the respective molecules, by substituting, into the independent variables in the equation, values for the electric signals from the measuring element and values for the electric signals from the heating element.

The electric power generating system and gas measuring system in the present disclosure may evaluate the number of atoms that form molecules included in a gas.

According to an embodiment, a gas measuring system includes a measuring element being in contact with a gas and a heating element producing heat, a measuring unit measuring a value for an electric signal from the temperature measuring element and a value for an electric signal from the heating element. The system also includes an equation storage device storing an equation including independent variables that represent electric signals from the temperature measuring element and the heating element, and a dependent variable that represents the sum of the products of the respective numbers of atoms forming the molecules in the gas, the atomic weights, and the volumetric ratios of the respective molecules. The system further includes a calculator calculating a value that represents the sum of the products of the respective numbers of the atoms, the atomic weights, and the volumetric ratios of the respective molecules.

Further advantages, features, aspects and details are evident from the dependent claims, the description and the drawings.

Brief Description of the Drawings

FIG. 1 is a first perspective view of a microchip as set forth in an example according to the present disclosure.
FIG. 2 is a cross-sectional diagram, viewed from the direction of the section II-II in FIG. 1, of the microchip illustrated in FIG. 1, as set forth in the example according to the present disclosure.
FIG. 3 is a second perspective view of a microchip as set forth in the example according to the present disclosure.
FIG. 4 is a cross-sectional diagram, viewed from the direction of the section IV-IV of the microchip illustrated in FIG. 3, as set forth in the example according to the present disclosure.
FIG. 5 is a circuit diagram relating to a heating element according to the example according to the present disclosure.
FIG. 6 is a circuit diagram relating to a temperature measuring element according to the example according to the present disclosure.
FIG. 7 is a graph illustrating the relationship between the temperature of the heating element and the radiation coefficient of the gas in the example according to the present disclosure.
FIG. 8 is a first schematic diagram of a gas measuring system as set forth in the example according to the present disclosure.
FIG. 9 is a second schematic diagram of a gas measuring system as set forth in the example according to the present disclosure.
FIG. 10 is a flowchart illustrating a method for generating an equation as set forth in the example according to the present disclosure.
FIG. 11 is a flowchart illustrating a method for measuring a gas as set forth in the example according to the present disclosure.
FIG. 12 is a graph showing the errors from the true values of measured values relating to another example according to the present disclosure.
FIG. 13 is a graph showing the errors from the true values of measured values relating to yet another example according to the present disclosure.
FIG. 14 is a graph showing the errors from the true values of measured values relating to yet a further example according to the present disclosure.
FIG. 15 is a schematic diagram of an electric power generating system as set forth in yet another further example according to the present disclosure.
FIG. 16 is a graph illustrating the relationship between the gas thermal conductivity and heat loss coefficient in yet still another further example according to the present disclosure.

Detailed Description

Examples of the present disclosure are described below. In the descriptions of the drawings below, identical or similar components are indicated by identical or similar codes. Note that the diagrams are schematic. Consequently, specific measurements should be evaluated in light of the descriptions below. Furthermore, even within these drawings there may, of course, be portions having differing dimensional relationships and proportions.

(Example)

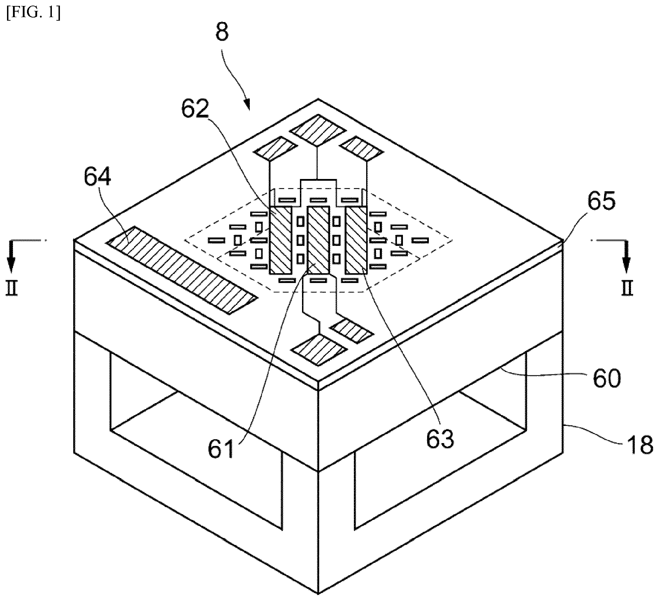

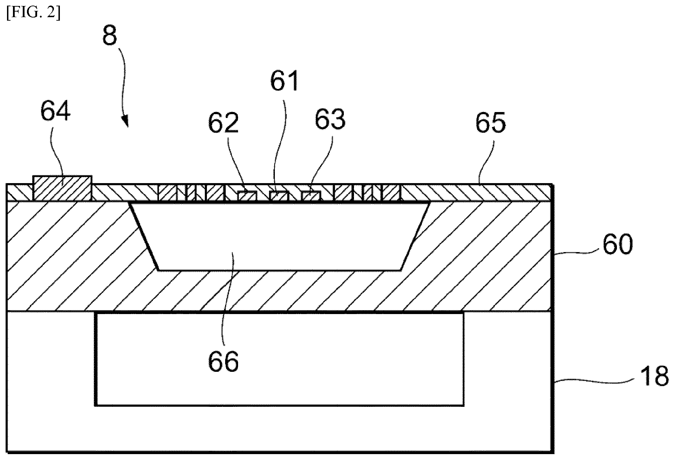

First, a microchip 8 that is used in a gas measuring system as set forth in an example is described in reference to FIG. 1, which is a perspective diagram, and FIG. 2, which is a cross-sectional diagram that is viewed from the direction of the section II-II. The microchip 8 comprises a substrate 60, which is provided with a cavity 66, and an insulating layer 65, which is disposed so as to cover the cavity 66 on the substrate 60. The thickness of the substrate 60 is, for example, 0.5 mm. The length and width dimensions of the substrate 60 are, for example, 1.5 mm each. The portion of the insulating layer 65 that covers the cavity 66 forms a thermally insulating diaphragm. The microchip 8 further comprises a heating element 61 that is provided on a portion of the diaphragm of the insulating layer 65, a first temperature measuring element 62 and a second temperature measuring element 63 provided in a portion of the diaphragm of the insulating layer 65 so that the heating element 61 is interposed therebetween, and a temperature maintaining element 64 that is provided on the substrate 60.

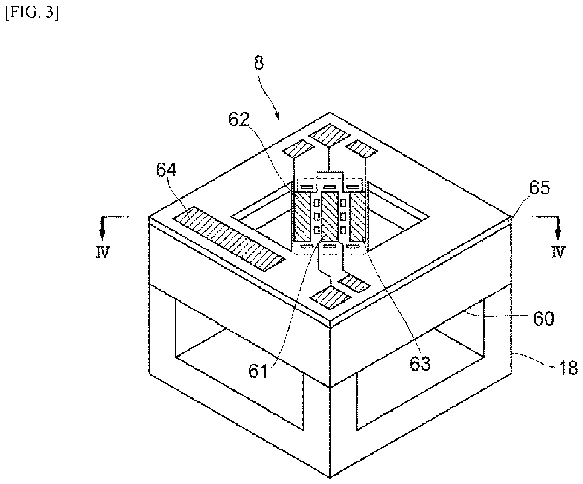

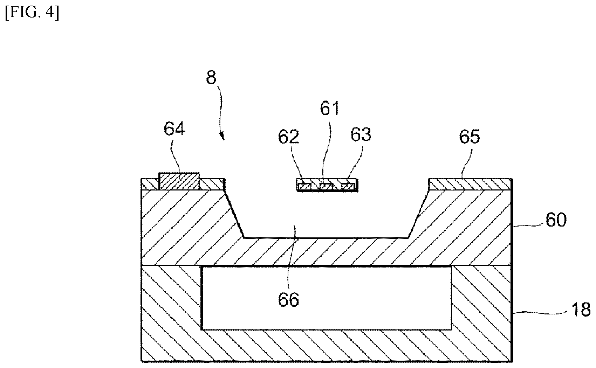

A plurality of holes is provided in the diaphragm. The provision of the plurality of holes in the diaphragm expedites the exchange of gases within the cavity 66. Conversely, the insulating layer 65, as illustrated in FIG. 3 and in FIG. 4, which is a cross-sectional diagram when viewed in the direction of the section IV-IV, may be disposed on the substrate 60 so as to cover the cavity 66 in the form of a bridge. This also exposes the inside of the cavity 66, expediting the exchange of gases within the cavity 66.

The heating element 61 is disposed in the center of the portion of the diaphragm of the insulating layer 65 that covers the cavity 66. The heating element 61 is, for example, a resistor, and produces heat through the supply of electric power thereto, to heat the ambient gas that contacts the heating element 61. The first temperature measuring element 62 and the second temperature measuring element 63 are electrical elements that are, for example, passive elements such as resistors, and output electric signals that are dependent on the gas temperatures of the surrounding gases. An example of use of the output signal of the first temperature measuring element 62 is explained below, but there is no limitation thereto, but rather, for example, an average value of the output signal from the first temperature measuring element 62 and the output signal of the second temperature measuring element 63 may be used as the output signal of the temperature measuring elements.

The temperature maintaining element 64 is, for example, a resistor, to which electricity is applied to produce heat, to maintain the substrate 60 at a constant temperature. Silicon (Si), or the like, may be used as the material for the substrate 60. Silicon dioxide ($SiO_2$), or the like, may be used as the material for the insulating layer 65. The cavity 66 may be formed through anisotropic etching, or the like. Furthermore, platinum (Pt) or the like may be used as the material for the first temperature measuring element 62, the second temperature measuring element 63, and the temperature maintaining element 64, and they may be formed through a lithographic method, or the like. Moreover, the heating element 61, the first temperature measuring element 62, and the second temperature measuring element 63 may be formed from the same member.

The microchip 8 is secured to a pipe, or the like, in which flows the ambient gas, through, for example, a thermally insulating member that is disposed on the bottom face of the microchip 8. Securing the microchip 8 through a thermally insulating member 18 within a pipe makes the temperature of the microchip 8 less susceptible to temperature variations of the inner wall of the pipe. The thermal conductivity of the insulating member 18, made from glass, or the like, is, for example, no more than 1.0 W/(m·K).

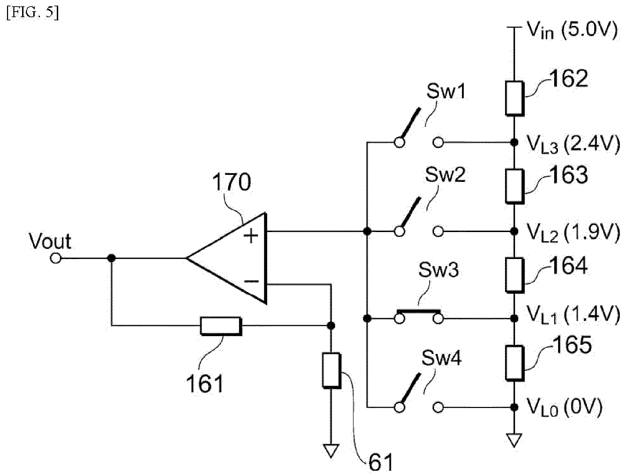

As illustrated in FIG. 5, one end of the heating element 61 is connected electrically to a - input terminal of an operational amplifier 170, for example, with the other end grounded. A resistive element 161 is connected, in parallel, to the - input terminal and the output terminal of the operational amplifier 170. The + input terminal of the operational amplifier 170 is connected electrically between a resistive element 162 and a resistive element 163, which are connected in series, between the resistive element 163 and a resistive element 164, which are connected in series, between the resistive element 164 and a resistive element 165, which are connected in series, or between the resistive element 165 and a ground terminal. The appropriate selection of the resistance values for each of the resistive elements 162 through 165 produces a voltage $V_{L3}$ of, for example, 2.4 V between the resistive element 163 and 162 when a voltage Vin of, for example, 5.0 V is applied to one end of the resistive element 162. Additionally, a voltage $V_{L2}$ of, for example, 1.9 V is produced between the resistive element 164 and the resistive element 163, and a voltage $V_{L1}$ of, for example, 1.4 V is produced between the resistive element 165 and the resistive element 164.

A switch SW1 is connected between the + input terminal of the operational amplifier 170 and a node between the resistive element 162 and the resistive element 163, and a switch SW2 is connected between the + input terminal of the operational amplifier 170 and a node between the resistive element 163 and the resistive element 164. Furthermore, a switch SW3 is provided between the + input terminal of the operational amplifier 170 and a node between the resistive element 164 and the resistive element 165, and a switch SW4 is provided between the + input terminal of the operational amplifier 170 and a node between the resistive element 165 and a ground terminal.

When applying the voltage $V_{L3}$ of 2.4 V to the + input terminal of the operational amplifier 170, only switch SW1 is turned ON, and switches SW2, SW3, and SW4 are turned OFF. When applying the voltage $V_{L2}$ of 1.9 V to the + input terminal of the operational amplifier 170, only switch SW2 is turned ON, and switches SW1, SW3, and SW4 are turned OFF. When applying the voltage $V_{L1}$ of 1.4 V to the + input terminal of the operational amplifier 170, only switch SW3 is turned ON, and switches SW1, SW2, and SW4 are turned OFF. When applying the voltage $V_{L0}$ of 0 V to the + input terminal of the operational amplifier 170, only switch SW4 is turned ON, and switches SW1, SW2, and SW3 are turned OFF. Consequently, 0 V or any of three levels of voltages can be applied to the + input terminal of the operational amplifier 170 through turning the switches SW1, SW2, SW3, and SW4 ON and OFF. Because of this, the applied voltages, which determine the heat producing temperature of the heating element 61, can be set to three different levels through opening and closing the switches SW1, SW2, SW3, and SW4.

Here, the temperature of the heating element 61 when the 1.4 V voltage $V_{L1}$ is applied to the + input terminal of the operational amp defined as $T_{H1}$. Additionally, the temperature of the heating element 61 when the 1.9 V voltage $V_{L2}$ is applied to the input terminal of the operational amplifier 170 is defined as $T_{H2}$, and the temperature of the heating element 61 when the 2.4 V voltage $V_{L3}$ is applied to the + input terminal of the operational amplifier 170 is defined as $T_{H3}$.

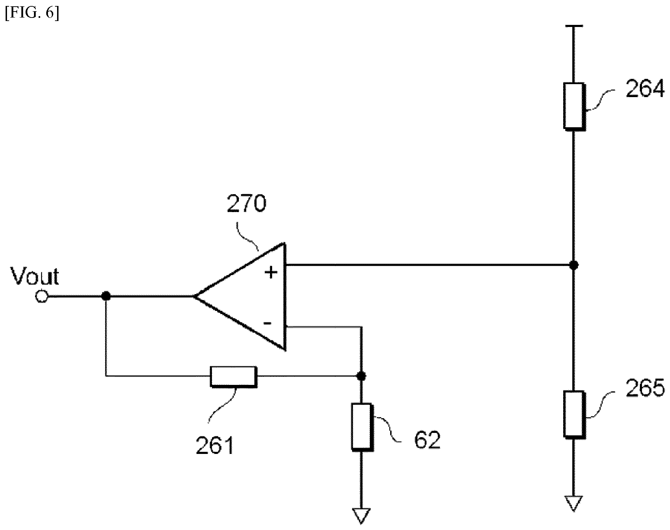

As illustrated in FIG. 6, one end of the first temperature measuring element 62 is connected electrically to a - input terminal of an operational amplifier 270, for example, with the other end grounded. A resistive element 261 is connected, in parallel, to the - input terminal and the output terminal of the operational amplifier 270. The + input terminal of the operational amplifier 270 is connected electrically to between a resistive element 264 and a resistive element 265 that are connected in series. This causes a weak voltage of about 0.3 V to be applied to the first temperature measuring element 62.

The resistance value of the heating element 61 illustrated in FIG. 1 and FIG. 2 varies depending on the temperature of the heating element 61. The relationship between the temperature $T_H$ of the heating element 61 and the resistance value $R_H$ of the heating element 61 is given through Equation (1), below:

$$R_H = R_{H\_STD} \times [1 + \alpha_H (T_H - T_{H\_STD}) + \beta_H (T_H - T_{H\_STD})^2] \ldots (1)$$

Here $T_{H\_STD}$ indicates a standard temperature for the heating element 61 of, for example, 20°C. $R_{H\_STD}$ indicates the resistance value of the heating element 61 measured in advance at the standards temperature of $T_{H\_STD}$. $\alpha_H$ indicates a first-order resistance temperature coefficient. $\beta_H$ indicates a second-order resistance temperature coefficient.

The resistance value $R_H$ of the heating element 61 is given by Equation (2), below, from the driving power $P_H$ of the heating element 61 and the current $I_H$ that flows through the heating element 61.

$$R_H = P_H / I_H^2 \ldots (2)$$

Conversely, the resistance value $R_H$ of the heating element 61 is given by Equation (3), below, from the voltage $V_H$ applied to the heating element 61 and the current $I_H$ that flows through the heating element 61.

$$R_H = V_H / I_H \ldots (3)$$

Here the temperature $T_H$ of the heating element 61 reaches a thermal equilibrium and stabilizes between the heating element 61 and the ambient gas. Note that this "thermal equilibrium" refers to a state wherein there is a balance between the heat production by the heating element 61 and the heat dissipation from the heating element 61 into the ambient gas. As shown in Equation (4), below, the driving power $P_H$ of the heating element 61 in the state of thermal equilibrium is divided by the difference $\Delta T_H$ between the temperature $T_H$ of the heat-producing element 61 and the temperature $T_I$ of the ambient gas, to produce the heat-dissipating factor $M_I$ of the ambient gas. Note that the units for the radiation coefficient $M_I$ are, for example, W/°C.

$$M_I = P_H / (T_H - T_I) = P_H / \Delta T_H \ldots (4)$$

From Equation (1), above, the temperature $T_H$ of the heating element 61 is obtained through Equation (5), below:

$$T_H = (1 / 2\beta_H) \times [-\alpha_H + [\alpha_H^2 - 4\beta_H (1 - R_H / R_{H\_STD})]^{1/2}] + T_{H\_STD} \ldots (5)$$

Consequently, the difference $\Delta T_H$ between the temperature $T_H$ of the heat-producing element 61 and the temperature $T_I$ of the ambient gas is given by Equation (6), below:

$$\Delta T_H = (1 / 2\beta_H) \times [-\alpha_H + [\alpha_H^2 - 4\beta_H (1 - R_H / R_{H\_STD})]^{1/2}] + T_{H\_STD} - T_I \ldots (6)$$

The temperature $T_I$ of the ambient gas temperature $T_I$ is approximated by the temperature $T_I$ of the first temperature measuring element 62 when power is applied to the extent that it does not produce heat itself. The relationship between the temperature $T_I$ of the first temperature measuring element 62 and the resistance value $R_I$ of the first temperature measuring element 62 is given by Equation (7), below:

$$R_I = R_{I\_STD} \times [1 + \alpha_I (T_I - T_{I\_STD}) + \beta_I (T_I - T_{I\_STD})^2] \ldots (7)$$

Here $T_{I\_STD}$ indicates a standard temperature for the first temperature measuring element 62 of, for example, 20°C. $R_{I\_STD}$ indicates the resistance value of the first temperature measuring element 62, measured in advance at the standard temperature of $T_{I\_STD}$. $A_I$ indicates a first-order resistance temperature coefficient. $B_I$ indicates a second-order resistance temperature coefficient. Through Equation (7), above, the temperature $T_I$ of the first temperature measuring element 62 is given by Equation (8), below:

$$T_I = (1 / 2\beta_I) \times [-\alpha_I + [\alpha_I^2 - 4\beta_I (1 - R_I / R_{I\_STD})]^{1/2}] + T_{I\_STD} \ldots (8)$$

Consequently, the radiation coefficient $M_I$ of the ambient gas is given by Equation (9), below.

$$M_I = P_H / \Delta T_H$$
$$= P_H / [ (1 / 2\beta_H) [-\alpha_H + [ \alpha_H^2 - 4\beta_H (1 - R_H / R_{H\_STD}) ]^{1/2}]$$
$$+ T_{H\_STD} - (1 / 2\beta_I) [ -\alpha_I + [ \alpha_I^2 - 4\beta_I (1 - R_I / R_{I\_STD}) ]^{1/2}] - T_{I\_STD}] \ldots (9)$$

The electric current $I_H$ that flows in the heating element 61 and the driving power $P_H$ or the voltage $V_H$ can be measured, and thus the resistance value $R_H$ of the heating element 61 can be calculated from Equation (2) or Equation (3), above. Similarly, it is also possible to calculate the resistance value $R_I$ of the first temperature measuring element 62. Consequently, the radiation coefficient $M_I$ of the ambient gas can be calculated from Equation (9), above, using the microchip 8.

Note that holding the temperature of the substrate 60 constant, using the temperature maintaining element 64, causes the temperature of the ambient gas in the vicinity of the microchip 8, prior to heating by the heating element 61, to approximate the constant temperature of the substrate 60. This suppresses the variation in the temperature of the ambient gas prior to heating by the heating element 61. Further heating, by the heating element 61, the ambient gas for which the temperature variation had been controlled makes it possible to calculate the radiation coefficient $M_I$ with greater accuracy.

Here the ambient gas is a mixed gas, where the mixed gas is assumed to comprise four gas components: gas A, gas B, gas C, and gas D. The total of the volume fraction $V_A$ of the gas A, the volume fraction $V_B$ of the gas B, the volume fraction $V_C$ of the gas C, and the volume fraction $V_D$ of the gas D, as obtained by Equation (10), below, is 1.

$$V_A + V_B + V_C + V_D = 1 \ldots (10)$$

When the per-unit-volume calorific value of gas A is defined as $K_A$, the per-unit-volume calorific value of gas B is defined as $K_B$, the per-unit-volume calorific value of gas C is defined as $K_C$, and the per-unit-volume calorific value of gas D is defined as $K_D$, then the per-unit-volume calorific value Q of mixed gas is obtained by summing the products of the volume fractions of the individual gas components and the per-unit-volume calorific values of the individual gas components. Consequently, the per-unit-volume calorific value Q of the mixed gas is given by Equation (11), below. Note that the units for the per-unit-volume calorific values are, for example, MJ/m3.

$$Q = K_A \times V_A + K_B \times V_B + K_C \times V_C + K_D \times V_D \ldots (11)$$

Moreover, when the per-unit-volume calorific value due to the hydrogen atoms that structure the gas A is defined as $K_{AH}$, the per-unit-volume calorific value due to the hydrogen atoms that structure the gas B is defined as $K_{BH}$, the per-unit-volume calorific value due to the hydrogen atoms that structure the gas C is defined as $K_{CH}$, and the per-unit-volume calorific value due to the hydrogen atoms that structure the gas D is defined as $K_{DH}$, then the per-unit-volume calorific value $Q_H$ due to the hydrogen atoms that structure the molecules included in the mixed gas is obtained by summing the products of the volume fractions of the individual gas components and the per-unit-volume calorific values due to the hydrogen atoms that structure the individual gas components. Consequently, the per-unit-volume calorific value $Q_H$ due to the hydrogen atoms that structure the molecules included in the mixed gas is given by Equation (12), below.

$$Q_H = K_{AH} \times V_A + K_{BH} \times V_B + K_{CH} \times V_C + K_{DH} \times V_D \ldots (12)$$

Moreover, when the heat-dissipating factor of gas A is defined as $M_A$, the heat-dissipating factor of gas B is defined as $M_B$, the heat-dissipating factor of gas C is defined as $M_C$, and the heat-dissipating factor of gas D is defined as $M_D$, then the heat-dissipating factor of the mixed gas $M_I$ is given by summing the products of the volume fractions of the individual gas components and the heat-dissipating factors of the individual gas components. Consequently, the radiation coefficient $M_I$ of the mixed gas is given by Equation (13), below.

$$M_I = M_A \times V_A + M_B \times V_B + M_C \times V_C + M_D \times V_D \ldots (13)$$

Moreover, because the radiation coefficient of the gas is dependent on the temperature $T_H$ of the heating element 61, the radiation coefficient $M_I$ of the mixed gas is given by Equation (14) as a function of the temperature $T_H$ of the heating element 61:

$$M_I(T_H) = M_A(T_H) \times_A + M_B(T_H) \times V_B + M_C(T_H) \times V_C + M_D(T_H) \times V_D \ldots (14)$$

Consequently, when the temperature of the heating element 61 is $T_{H1}$, then the radiation coefficient $M_{I1}(T_{H1})$ of the mixed gas is given by Equation (15), below. Moreover, when the temperature of the heating element 61 is $T_{H2}$, then the radiation coefficient $M_{I2}(T_{H2})$ of the mixed gas is given by Equation (16), below, and when the temperature of the heating element 61 is $T_{H3}$, then the radiation coefficient $M_{I3}(T_{H3})$ of the mixed gas is given by Equation (17), below.

$$M_{I1}(T_{H1}) = M_A(T_{H1}) \times V_A + M_B(T_{H1}) \times V_B + M_C(T_{H1}) \times V_C + M_D(T_{H1}) \times V_D \ldots (15)$$

$$M_{I2}(T_{H2}) = M_A(T_{H2}) \times V_A + M_B(T_{H2}) \times V_B + M_C(T_{H2}) \times V_C + M_D(T_{H2}) \times V_D \ldots (16)$$

$$M_{I3}(T_{H3}) = M_A(T_{H3}) \times V_A + M_B(T_{H3}) \times V_B + M_C(T_{H3}) \times V_C + M_D(T_{H3}) \times V_D \ldots (17)$$

If here the radiation coefficients $M_A(T_H)$, $M_B(T_H)$, $M_C(T_H)$, and $M_D(T_H)$ of the individual gas components are non-linear in respect to the temperature $T_H$ of the heating element 61, then the Equations (15) through (17), above, have linearly independent relationships. Moreover, even if the radiation coefficients $M_A(T_H)$, $M_B(T_H)$, $M_C(T_H)$, and $M_D(T_H)$ of the individual gas components are linear in respect to the temperature $T_H$ of the heating element 61, if the rates of change of the radiation coefficients $M_A(T_H)$, $M_B(T_H)$, $M_C(T_H)$, and $M_D(T_H)$ of the individual gas components are non-linear in respect to the temperature $T_H$ of the heating element 61 the Equations (15) through (17), above, have linearly independent relationships. Moreover, if Equations (15) through (17) have a linearly independent relationship, then Equation (10) and Equations (15) through (17) have a linearly independent relationship.

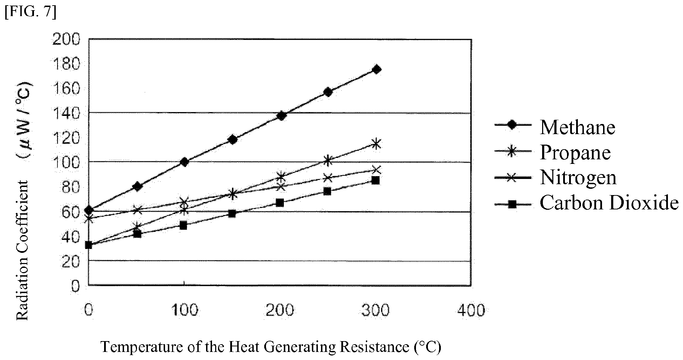

FIG. 7 is a graph showing the relationships of the radiation coefficients of methane (CH4), propane ($C_3H_8$), nitrogen ($N_2$), and carbon dioxide ($CO_2$), which are included in natural gas or utility gas, to the temperature of the heating element 61 which is a heat producing resistance. The radiation coefficients of each of these components (methane ($CH_4$), propane ($C_3H_8$), nitrogen ($N_2$), and carbon dioxide ($CO_2$)) are linear in respect to the temperature of the heating element 61. However, the respective rates of change of the radiation coefficients in respect to the temperature of the heating element 61 are different for methane ($CH_4$), propane ($C_3H_8$), nitrogen ($N_2$), and carbon dioxide ($CO_2$). Consequently, Equations (15) through (17), above, are linearly independent if the gas components that comprise the mixed

gas are methane ($CH_4$), propane ($C_3H_8$), nitrogen ($N_2$), and carbon dioxide ($CO_2$).

The values for the radiation coefficients $M_A(T_{H1})$, $M_B(T_{H1})$, $M_C(T_{H1})$, $M_D(T_{H1})$, $M_A(T_{H2})$, $M_B(T_{H2})$, $M_C(T_{H2})$, $M_D(T_{H2})$, $M_A(T_{H3})$, $M_B(T_{H3})$, $M_C(T_{H3})$, $M_D(T_{H3})$ for the individual gas components in Equation (15) through Equation (17) can be obtained in advance through measurements, or the like. Consequently, when the system of simultaneous equations of Equation (10) and Equation (15) through Equation (17) is solved, the volume fraction $V_A$ of the gas A, the volume fraction $V_B$ of the gas B, the volume fraction $V_C$ of the gas C, and the volume fraction $V_D$ of the gas D, respectively, are obtained as functions of the heat-dissipating factors $M_{H1}(T_{H1})$, $M_{I2}(T_{H2})$, and $M_{I3}(T_{H3})$ of the mixed gas, as indicated in Equation (18) through Equation (21), below. Note that in Equations (18) through (21), below, $f_n$, where n is a non-negative integer, is a code representing a function:

$$V_A = f_1[M_{I1}(T_{H1}), M_{I2}(T_{H2}), M_{I3}(T_{H3})] \ldots(18)$$

$$V_B = f_2[M_{I1}(T_{H1}), M_{I2}(T_{H2}), M_{I3}(T_{H3})] \ldots(19)$$

$$V_C = f_3[M_{I1}(T_{H1}), M_{I2}(T_{H2}), M_{I3}(T_{H3})] \ldots(20)$$

$$V_D = f_4[M_{I1}(T_{H1}), M_{I2}(T_{H2}), M_{I3}(T_{H3})] \ldots(21)$$

Here Equation (22), below, is obtained through substituting Equation (18) through (21) into Equation (11), above.

$$Q = K_A \times V_A + K_B \times V_B + K_C \times V_C + K_D \times V_D = K_A \times f1[M_{I1}(T_{H1}), M_{I2}(T_{H2}), M_{I3}(T_{H3})]$$
$$+ K_B \times f_2[M_{I1}(T_{H1}), M_{I2}(T_{H2}), M_{I3}(T_{H3})] + K_C \times f_3[M_{I1}(T_{H1}), M_{I2}(T_{H2}), M_{I3}(T_{H3})]$$
$$+ K_D \times f_4[M_{I1}(T_{H1}), M_{I2}(T_{H2}), M_{I3}(T_{H3})] \ldots(22)$$

As shown in Equation (22), above, the per-unit-volume calorific value Q is obtained as an equation which has, as variables, the radiation coefficients $M_{I1}(T_{H1})$, $M_{I2}(T_{H2})$, and $M_{I3}(T_{H3})$ of the mixed gas when the temperatures of the heating element 61 are $T_{H1}$, $T_{H2}$, and $T_{H3}$. Consequently, the calorific value Q of the mixed gas is given by Equation (23), below, where $g_1$ is a code representing a function.

$$Q = g_1[M_{I1}(T_{H1}), M_{I2}(T_{H2}), M_{I3}(T_{H3})] \ldots(23)$$

Moreover, according to Equation (12) and Equation (18) through Equation (21), above, the per-unit-volume calorific value $Q_H$ due to the hydrogen atoms that structure the molecules included in the mixed gas is given by Equation (24), below, where $g_2$ is a code representing a function.

$$Q_H = g_2[M_{I1}(T_{H1}), M_{I2}(T_{H2}), M_{I3}(T_{H3})] \ldots(24)$$

Consequently, the inventors discovered that, for a mixed gas comprising a gas A, a gas D, a gas C, and a gas D, wherein the volume fraction $V_A$ of the gas A, the volume fraction $V_B$ of the gas B, the volume fraction $V_C$ of the gas C, and the volume fraction $V_D$ of the gas D, are unknown, it is possible to calculate easily the per-unit-volume calorific value due to the hydrogen atoms that structure the molecules included in the mixed gas to be measured if Equation (24) is obtained in advance. Specifically, it is possible to calculate uniquely the calorific value $Q_H$ due to the hydrogen atoms that structure the molecules included in the mixed gas to be measured, through measuring the radiation coefficients $M_{I1}(T_{H1})$, $M_{I2}(T_{H2})$, and $M_{I3}(T_{H3})$ for the mixed gas to be measured, at the heat producing temperatures of $T_{H1}$, $T_{H2}$, and $T_{H3}$ of the heating element 61 and then substituting, into Equation (24).

Moreover, the radiation coefficient $M_I$ of the mixed gas, as indicated in Equation (9), above, depends on the resistance value $R_H$ of the heating element 61 and on the resistance value $R_I$ of the first temperature measuring element 62. Given this, the inventors discovered that the per-unit-volume calorific value $Q_H$ due to the hydrogen atoms that structure the molecules included in a mixed gas can also be obtained from an equation having, as variables, the resistances $R_{H1}(T_{H1})$, $R_{H2}(T_{H2})$, and $R_{H3}(T_{H3})$ of the heating element 61 when the temperatures of the heating element 61 are $T_{H1}$, $T_{H2}$, and $T_{H3}$, and the resistance value $R_I$ of the first temperature measuring element 62 that is in contact with the mixed gas as shown in Equation (25), below, where $h_1$ is a code representing a function.

$$Q_H = h_1[R_{H1}(T_{H1}), R_{H2}(T_{H2}), R_{H3}(T_{H3}), Ri] \ldots(25)$$

Given this, the calorific value $Q_H$ due to the hydrogen atoms that structure the molecules included in the mixed gas to be measured can be calculated uniquely also by substituting, into Equation 25, the resistances $R_{H1}(T_{H1})$, $R_{H2}(T_{H2})$, and $R_{H3}(T_{H3})$ of the heating element 61 when the heat producing temperatures of the heating element 61, which is in contact with the mixed gas to be measured, are $T_{H1}$, $T_{H2}$, and $T_{H3}$, and the resistance value $R_I$ of the first temperature measuring element 62 that is in contact with the mixed gas.

Moreover, the per-unit-volume calorific value $Q_H$ due to the hydrogen atoms that structure the molecules included in a mixed gas can also be obtained from an equation having, as variables, the currents $I_{H1}(T_{H1})$, $I_{H2}(T_{H2})$, and $I_{H3}(T_{H3})$ of the heating element 61 when the temperatures of the heating element 61 are $T_{H1}$, $T_{H2}$, and $T_{H3}$, and the current $I_I$ of the first temperature measuring element 62 that is in contact with the mixed gas as shown in Equation (26), below, where $h_2$ is a code representing a function.

$$Q_H = h_2[I_{H1}(T_{H1}), I_{H2}(T_{H2}), I_{H3}(T_{H3}), I_I] \ldots(26)$$

Conversely, the per-unit-volume calorific value $Q_H$ due to the hydrogen atoms that structure the molecules included in a mixed gas can also be obtained from an equation having, as variables, the voltages $V_{H1}(T_{H1})$, $V_{H2}(T_{H2})$, and $V_{H3}(T_{H3})$ applied to the heating element 61 when the temperatures of the heating element 61 are $T_{H1}$, $T_{H2}$, and $T_{H3}$, and the voltage $V_I$ that is applied to the first temperature measuring element 62 that is in contact with the mixed gas as shown in Equation (27), below, where $h_3$ is a code representing a function.

$$Q_H = h_3[V_{H1}(T_{H1}), V_{H2}(T_{H2}), V_{H3}(T_{H3}), V_I] \ldots(27)$$

Conversely, the per-unit-volume calorific value due to the hydrogen atoms that structure the molecules included in a mixed gas can also be obtained from an equation having, as variables, the output voltages $AD_{H1}(T_{H1})$, $AD_{H2}(T_{H2})$, and $AD_{H3}(T_{H3})$ of analog-digital converting circuits (hereinafter termed "A/D converting circuits") that are connected to the heating element 61 when the temperatures of the heating element 61 are $T_{H1}$, $T_{H2}$, and $T_{H3}$, and the output voltage $AD_I$ of an A/D converting circuit that is connected to the first temperature measuring element 62 that is in contact with the mixed gas, as shown in Equation (28), below, where $h_4$ is a code representing a function.

$$Q_H = h_4[AD_{H1}(T_{H1}), AD_{H2}(T_{H2}), AD_{H3}(T_{H3}), AD_I] \ldots(28)$$

Consequently, the per-unit-volume calorific value $Q_H$ due to the hydrogen atoms that structure the molecules included in a mixed gas can also be obtained from an equation having, as variables, electric signals $S_{H1}(T_{H1})$, $S_{H2}(T_{H2})$, and $S_{H3}(T_{H3})$ of the heating element 61 when the temperatures of the heating element 61 are $T_{H1}$, $T_{H2}$, and $T_{H3}$, and an electric signal $S_I$ of the first temperature measuring element 62 that is in contact with the mixed gas as shown in Equation (29), below, where $h_5$ is a code representing a function.

$$Q_H = h_5[S_{H1}(T_{H1}), S_{H2}(T_{H2}), S_{H3}(T_{H3}), S_I] \ldots(29)$$

Next, the per-unit-volume calorific value $Q_H$ due to the hydrogen atoms that structure the molecules included the mixed gas is correlated to the sum of the products of the respective numbers $N_H$ of hydrogen atoms that structure the molecules included in the mixed gas, the atomic weight of hydrogen (for example, 1.00794), and the volumetric ratios of the respective molecules in the mixed gas. Consequently, based on Equation (29), above, the sum $G_H$ of the products of the respective numbers $N_H$ of hydrogen atoms that structure the molecules included in the mixed gas, the atomic weight of hydrogen, and the volumetric ratios of the respective molecules is given by Equation (30), below, where $h_6$ is a code representing a function.

$$G_H = h_6[S_{H1}(T_{H1}), S_{H2}(T_{H2}), S_{H3}(T_{H3}), S_I] \ldots(30)$$

Note that the gas components of the mixed gas are not limited to four different components. For example, if the mixed gas comprises n types of gas components, then first a formula, given by Equation (31), below, is obtained using, as variables, the electric signals from the heating element 61 $S_{H1}(T_{H1})$, $S_{H2}(T_{H2})$, $S_{H3}(T_{H3})$, ..., $S_{Hn-1}(T_{Hn-1})$ at at least n-1 different the heat producing temperatures $T_{H1}$, $T_{H2}$, $T_{H3}$,..., $T_{Hn-1}$, and the electric signal $S_I$ from the first temperature measuring element 62. Given this, the per-unit-volume calorific value the sum $G_H$ of the products of the respective numbers $N_H$ of hydrogen atoms that structure the molecules included in the mixed gas, the atomic weight of hydrogen, and the volumetric ratios of the respective molecules can be calculated uniquely by measuring the values of the electric signals $S_{H1}(T_{H1})$, $S_{H2}(T_{H2})$, $S_{H3}(T_{H3})$, ..., $S_{Hn-1}(T_{Hn-1})$ from the heating element 61, which contacts the mixed gas to be measured that comprises n different component gases for which the respective volume fractions are unknown, and the value of the electric signal $S_I$ from the first temperature measuring element 62, and then substituting into Equation (31).

$$G_H = h_{12}[S_{H1}(T_{H1}), S_{H2}(T_{H2}), S_{H3}(T_{H3}), \ldots, S_{Hn-1}(T_{Hn-1}), S_I]\ldots(31)$$

For example, let us assume that methane ($CH_4$) is included at 90 VOL%, ethane ($C_2H_6$) is included at 5 VOL%, propane ($C_4H_{10}$) is included at 1 VOL%, nitrogen ($N_2$) is included at 1 VOL%, and carbon dioxide gas ($CO_2$) is included at 2 VOL%. In this case, because the number of hydrogen atoms that structure the methane is 4, the product of the number of hydrogen atoms that structure the methane, the atomic weight of hydrogen, and the volumetric ratio of the methane is 4 x 1.00794 x 0.9 = 3.628584. Moreover, because the number of hydrogen atoms that structure the ethane is 6, the product of the number of hydrogen atoms that structure the ethane, the atomic weight of hydrogen, and the volumetric ratio of the ethane is 6 x 1.00794 x 0.05 = 0.302382. Moreover, because the number of hydrogen atoms that structure the propane is 8, the product of the number of hydrogen atoms that structure the propane, the atomic weight of hydrogen, and the volumetric ratio of the propane is 8 x 1.00794 x 0.01 = 0.0806352. Moreover, because the number of hydrogen atoms that structure the butane is 10, the product of the number of hydrogen atoms that structure the butane, the atomic weight of hydrogen, and the volumetric ratio of the butane is 10 x 1.00794 x 0.01 = 0.100794. The numbers of hydrogen atoms that structure the nitrogen and the carbon dioxide are zero.

Consequently, the sum of the product of the number of hydrogen atoms that structure the methane, the atomic weight of hydrogen, and the volumetric ratio of the methane, the product of the number of hydrogen atoms that structure the ethane, the atomic weight of hydrogen, and the volumetric ratio of the ethane, the product of the number of hydrogen atoms that structure the propane, the atomic weight of hydrogen, and the volumetric ratio of the propane, and the product of the number of hydrogen atoms that structure the butane, the atomic weight of hydrogen, and the volumetric ratio of the butane is 3.628584+0.302382+0.0806352+0.100794=4.1123952.

Note that if the mixed gas includes an alkane ($C_jH_{2j+2}$) other than methane ($CH_4$) and propane ($C_3H_8$), where j is a natural number, in addition to methane ($CH_4$) and propane ($C_3H_8$), then the alkane ($C_jH_{2j+2}$) other than methane ($CH_4$) and propane ($C_3H_8$) is seen as a mixture of methane ($CH_4$) and propane ($C_3H_8$), and there is no effect on the calculation in Equation (31). For example, as indicated in Equations (32) through (35), below, the calculation may be performed using Equation (31) by viewing ethane ($C_2H_6$), butane ($C_4H_{10}$), pentane ($C_5H_{12}$), and hexane ($C_6H_{14}$) as a mixture of methane ($CH_4$) and propane ($C_3H_8$), with each multiplied by the respective specific factors.

$$C_2H_6 = 0.5\ CH_4 + 0.5\ C_3H_8\ldots(32)$$

$$C_4H_{10} = -0.5\ CH_4 + 1.5\ C_3H_8\ldots(33)$$

$$C_5H_{12} = -1.0\ CH_4 + 2.0\ C_3H_8\ldots(34)$$

$$C_6H_{14} = -1.5\ CH_4 + 2.5\ C_3H_8\ldots(35)$$

Consequently, with z as a natural number, if a mixed gas comprising n types of gas components includes, as gas components, z types of alkanes ($C_jH_{2j+2}$) other than methane ($CH_4$) and propane ($C_3H_8$), in addition to methane ($CH_4$) and propane ($C_3H_8$), an equation may be calculated having, as variables, the electric signals $S_H$ from the heating element 61 at, at least, n - z-1 different heat producing temperatures, and the electric signal $S_I$ from the first temperature measuring element 62.

If the types of gas components in the mixed gas used in the calculation in Equation (31) are the same as the types of gas components of the mixed gas to be measured, then, of course, Equation (31) can be used. Furthermore, Equation (31) can also be used when the mixed gas to be measured comprises a number of gas components that is less than n, where the gas components of the less than n different types are included in the mixed gas that is used for calculating Equation (31). If, for example, the mixed gas used in calculating Equation (31) included four types of gas components, namely methane ($CH_4$), propane ($C_3H_8$), nitrogen ($N_2$) and carbon dioxide ($CO_2$), then even if the mixed gas to be measured includes only three different components, namely methane ($CH_4$), propane ($C_3H_8$), and carbon dioxide ($CO_2$), without containing the nitrogen ($N_2$), still Equation (31) can be used.

Furthermore, if the mixed gas used in calculating Equation (31) included methane ($CH_4$) and propane ($C_3H_8$) as gas components, Equation (31) could still be used even when the mixed gas to be measured includes an alkane ($C_jH_{2j+2}$) that is not included in the mixed gas that is used in calculating Equation (31). As described above, this is because an alkane ($C_jH_{2j+2}$) other than methane ($CH_4$) and propane ($C_3H_8$) can be viewed as a mixture of methane ($CH_4$) and propane ($C_3H_8$).

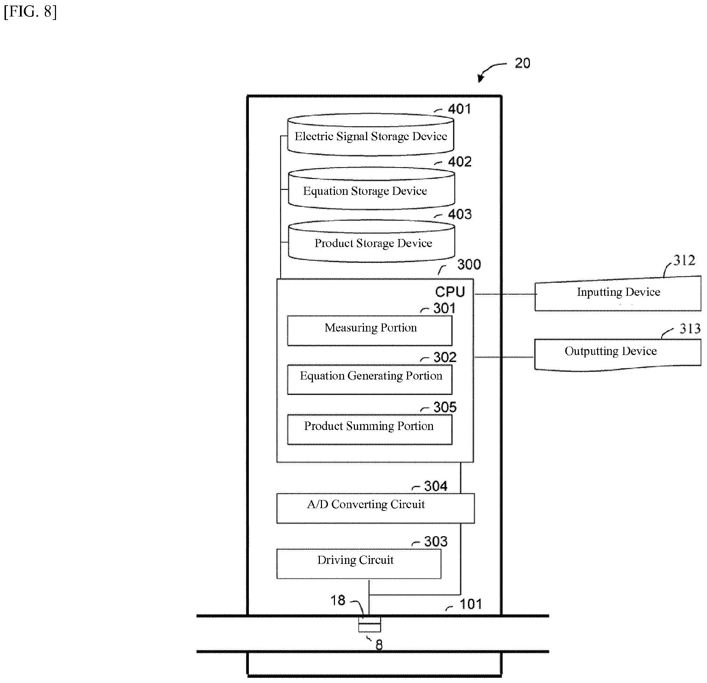

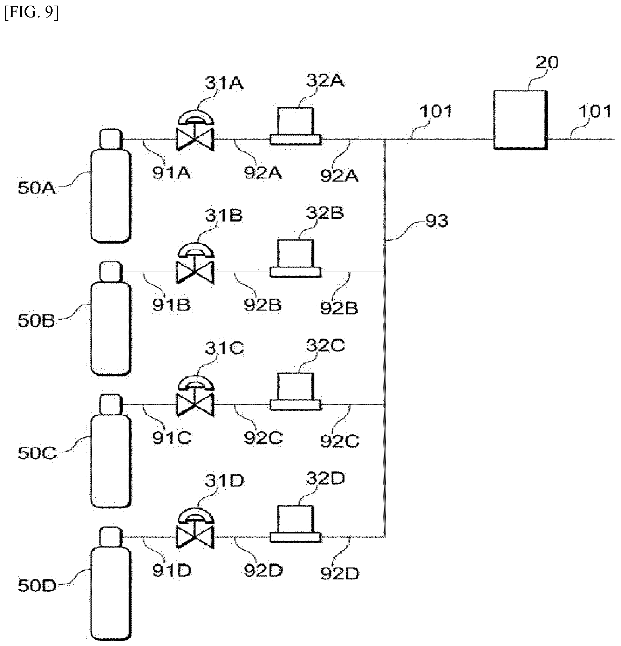

Here the gas measuring system 20 according to the example illustrated in FIG. 8 and FIG. 9 comprises: a pipe 101 through which flow each of the plurality of sample mixed gases; and, disposed within the pipe 101, a microchip 8 that includes the first temperature measuring element 62 and the heating element 61 for producing heat at a plurality of heat producing temperatures $T_H$, illustrated in FIG. 1. Note that the each of the sample mixed gases includes a plurality of types of gases. Moreover, the gas measuring system 20 illustrated in FIG. 8 comprises a measuring portion 301 for measuring the values of the electric signals $S_I$ from the first temperature measuring element 62, which are respectively dependent on the plurality of temperatures $T_I$ of the sample mixed gas, and the values of the electric signals $S_H$ from the heating element 61 at each of the respective heat producing temperatures $T_H$ of the plurality thereof. Moreover, the gas measuring system 20 comprises: an equation generating portion 302 for generating an equation, based the sum of the products of the respective numbers of hydrogen atoms $N_H$ that structure the molecules included in the gas, the atomic weight of hydrogen, and the volumetric ratios of the respective molecules of a plurality of sample mixed gases, the values for the electric signals $S_I$ from the first temperature measuring element 62, and the plurality of values for the electric signals from the heating element 61 at the plurality of heat producing temperatures, having an electric signal $S_I$ from the first temperature measuring element 62 and the electric signals $S_H$ from the heating element 61 at the plurality of heat producing temperatures $T_H$ as independent variables, and having the sum of the products of the respective numbers of hydrogen atoms that structure the molecules included in the gas, the atomic weight of hydrogen, and the volumetric ratios of the respective molecules as the dependent variable.

When a four types of sample mixed gases, each having a different sum of the products of the respective numbers of hydrogen atoms $N_H$ that structure the molecules included in the gas, the atomic weight of hydrogen, and the volumetric ratios of the respective molecules, are used, then, as illustrated in FIG. 9, a first gas canister 50A for storing a first sample mixed gas, a second gas canister 50B for storing a second sample mixed gas, a third gas canister 50C for storing a third sample mixed gas, and a fourth gas canister 50D for storing a fourth sample mixed gas are prepared. The first gas canister 50A is connected, through a pipe 91A to a first gas pressure regulating device 31A for providing the first sample mixed gas from the first gas canister 50A, regulated to a low-pressure such as, for example, 0.2 MPa. Additionally, a first flow rate controlling device 32A is connected through a pipe 92A to the first gas pressure regulating device 31A. The first flow rate controlling device 32A controls the rate of flow of the first sample mixed gas that is fed into gas measuring system 20 through the pipes 92A and 101.

A second gas pressure regulating device 31B is connected through a pipe 91B to the second gas canister 50B. Additionally, a second flow rate controlling device 32B is connected through a pipe 92B to the second gas pressure regulating device 31B. The second flow rate controlling device 32B controls the rate of flow of the second sample mixed gas that is fed into gas measuring system 20 through the pipes 92B, 93, and 101.

A third gas pressure regulating device 31C is connected through a pipe 91C to the third gas canister 50C. Additionally, a third flow rate controlling device 32C is connected through a pipe 92C to the third gas pressure regulating device 31C. The third flow rate controlling device 32C controls the rate of flow of the third sample mixed gas that is fed into gas measuring system 20 through the pipes 92C, 93, and 101.

A fourth gas pressure regulating device $31_D$ is connected through a pipe 91D to the fourth gas canister 50D. Additionally, a fourth flow rate controlling device 32D is connected through a pipe 92D to the fourth gas pressure regulating device 31D. The fourth flow rate controlling device 32D controls the rate of flow of the fourth sample mixed gas that is fed into gas measuring system 20 through the pipes 92D, 93, and 101.

The first through fourth at sample mixed gases are each, for example, natural gas or utility gas. The first through fourth sample mixed gases each include four different gas components of, for example, methane ($CH_4$), propane ($C_3H_8$), nitrogen ($N_2$), and carbon dioxide ($CO_2$) at different volumetric ratios.

When a first sample mixed gas is provided to the pipe 101, illustrated in FIG. 8, the first temperature measuring element 62 of the microchip 8, illustrated in FIG. 1 and FIG. 2, outputs an electric signal $S_I$ that is dependent on the temperature of the first sample mixed gas. Following this, the heating element 61 applies sequentially driving powers $P_{H1}$, $P_{H2}$, and $P_{H3}$ from the driving circuit 303 illustrated in FIG. 8. When the driving powers $P_{H1}$, $P_{H2}$, and $P_{H3}$ are applied, the heating element 61 that is in contact with the first sample mixed gas produces sequentially heat at a temperature $T_{H1}$ of 100°C, a temperature $T_{H2}$ of 150°C, and a temperature $T_{H3}$ of 200°C, for example, to output an electric signal $S_{H1}$ ($T_{H1}$) at the heat producing temperature $T_{H1}$, an electric signal $S_{H2}$ ($T_{H2}$) at the heat producing temperature $T_{H2}$, and an electric signal $S_{H3}$ ($T_{H3}$) at the heat producing temperature $T_{H3}$.

After the removal of the first sample mixed gas from the pipe 101, the second through fourth sample mixed gases are provided sequentially through the pipe 101. When the second sample mixed gas is provided to the pipe 101, the first temperature measuring element 62 of the microchip 8, illustrated in FIG. 1 and FIG. 2, outputs an electric signal SI that is dependent on the temperature of the second sample mixed gas. Following this, the heating element 61, which is in contact with the second sample mixed gas, outputs an electric signal $S_{H1}$ ($T_{H1}$) at a heat producing temperature $T_{H1}$, an electric signal $S_{H2}$ ($T_{H2}$) at a heat producing temperature $T_{H2}$, and an electric signal $S_{H3}$ ($T_{H3}$) at a heat producing temperature $T_{H3}$.

When a third sample mixed gas is provided to the pipe 101, illustrated in FIG. 8, the first temperature measuring element 62 of the microchip 8, illustrated in FIG. 1 and FIG. 2, outputs an electric signal $S_I$ that is dependent on the temperature of the third sample mixed gas. Following this, the heating element 61, which is in contact with the third sample mixed gas, outputs an electric signal $S_{H1}$ ($T_{H1}$) at a heat producing temperature $T_{H1}$, an electric signal $S_{H2}$ ($T_{H2}$) at a heat producing temperature $T_{H2}$, and an electric signal $S_{H3}$ ($T_{H3}$) at a heat producing temperature $T_{H3}$.

When a fourth sample mixed gas is provided to the pipe 101, illustrated in FIG. 8, the first temperature measuring element 62 of the microchip 8, illustrated in FIG. 1 and FIG. 2, outputs an electric signal $S_I$ that is dependent on the temperature of the fourth sample mixed gas. Following this, the heating element 61, which is in contact with the fourth sample mixed gas, outputs an electric signal $S_{H1}$ ($T_{H1}$) at a heat producing temperature $T_{H1}$, an electric signal $S_{H2}$ ($T_{H2}$) at a heat producing temperature $T_{H2}$, and an electric signal $S_{H3}$ ($T_{H3}$) at a heat producing temperature $T_{H3}$.

Note that if there are n types of gas components in each of the sample mixed gases, the heating element 61 of the microchip 8, illustrated in FIG. 1 and FIG. 2, is caused to produce heat at at least n-1 different temperatures. However, as described above, an alkane ($C_jH_{2j+2}$) other than methane ($CH_4$) and propane ($C_3H_8$) can be viewed as a mixture of methane ($CH_4$) and propane ($C_3H_8$). Consequently, with z as a natural number, if a sample mixed gas comprising n types of gas components includes, as gas components, z types of alkanes ($C_jH_{2j+2}$) in addition to methane ($CH_4$) and propane ($C_3H_8$), the heating element 61 is caused to produce heat at n-z-1 different temperatures.

As illustrated in FIG. 8, the microchip 8 is connected to a central calculation processing device (CPU) 300 that includes the measuring portion 301. An electric signal storing device 401 is also connected to the CPU 300. The measuring portion 301 measures the value of the electric signal $S_I$ from the first temperature measuring element 62, and, from the heating element 61, the values of the electric signal $S_{H1}$ ($T_{H1}$) at the heat producing temperature $T_{H1}$, the electric signal $S_{H2}$ ($T_{H2}$) at the heat producing temperature $T_{H2}$, and the electric signal $S_{H3}$ ($T_{H3}$) at the heat producing temperature $T_{H3}$, and stores the measured values in the electric signal storage device 401.

Note that electric signal $S_I$ from the first temperature measuring element 62 may be the resistance value $R_I$ of the first temperature measuring element 62, the current $I_I$ flowing in the first temperature measuring element 62, the voltage $V_I$ applied to the first temperature measuring element 62, or the output signal $AD_I$ from the A/D converting circuit 304 that is connected to the first temperature measuring element 62. Similarly, the electric signal $S_H$ from the heating element 61 may be the resistance value $R_H$ of the heating element 61, the current $I_H$ flowing in the heating element 61, the voltage $V_H$ applied to the heating element 61, or the output signal $AD_H$ from the A/D converting circuit 304 that is connected to the heating element 61.

The equation generating portion 302 that is included in the CPU 300 collects, for example, a known value for the sum of the products of the respective numbers of hydrogen atoms $N_H$ that structure the molecules included in a first sample mixed gas, the atomic weight of hydrogen, and the volumetric ratios of the respective molecules, a known value for the sum of the products of the respective numbers of hydrogen atoms $N_H$ that structure the molecules included in a second sample mixed gas, the atomic weight of hydrogen, and the volumetric ratios of the respective molecules, a known value for the sum of the products of the respective numbers of hydrogen atoms $N_H$ that structure the molecules included in a third sample mixed gas, the atomic weight of hydrogen, and the volumetric ratios of the respective molecules, and a known value for the sum of the products of the respective numbers of hydrogen atoms $N_H$ that structure the molecules included in a fourth sample mixed gas, the atomic weight of hydrogen, and the volumetric ratios of the respective molecules, the plurality of measured values for the electric signals $S_I$ from the first temperature measuring element 62, and the plurality of measured values for the electric signals $S_{H1}$ ($T_{H1}$), $S_{H2}$ ($T_{H2}$), and $S_{H3}$ ($T_{H3}$) from the heating element 61. Moreover, the equation generating portion 302 calculates an equation, through multivariate statistics, based on the collected values for the sums of the products, the values of the electric signals $S_I$, and the values of the electric signals $S_H$, with the electric signal $S_I$ from the first temperature measuring element 62, and the electric signals $S_{H1}$($T_{H1}$), $S_{H2}$($T_{H2}$), and $S_{H3}$($T_{H3}$) from the heating element 61 as the independent variables and the sum of the products of the respective numbers of hydrogen atoms $N_H$ that structure the molecules included in the gas, the atomic weight of hydrogen, and the volumetric ratios of the respective molecules as the dependent variable.

Note that "multivariate statistics" includes support vector analysis disclosed in A. J. Smola and B. Scholkopf (eds.), "A Tutorial on Support Vector Regression" (NeuroCOLT Technical Report NC-TR-98-030), multiple linear regression analysis, the Fuzzy Quantification Theory Type II, disclosed in Japanese Unexamined Patent Application Publication H5-141999, and the like.

The gas measuring system 20 is further provided with an equation storage device 402, connected to the CPU 300. The equation storage device 402 stores the equation generated by the equation generating portion 302. An inputting device 312 and an outputting device 313 are also connected to the CPU 300. A keyboard, a pointing device such as a mouse, or the like, may be used as the inputting device 312. An image displaying device such as a liquid crystal display or a monitor, or a printer, or the like, may be used as the outputting device 313.

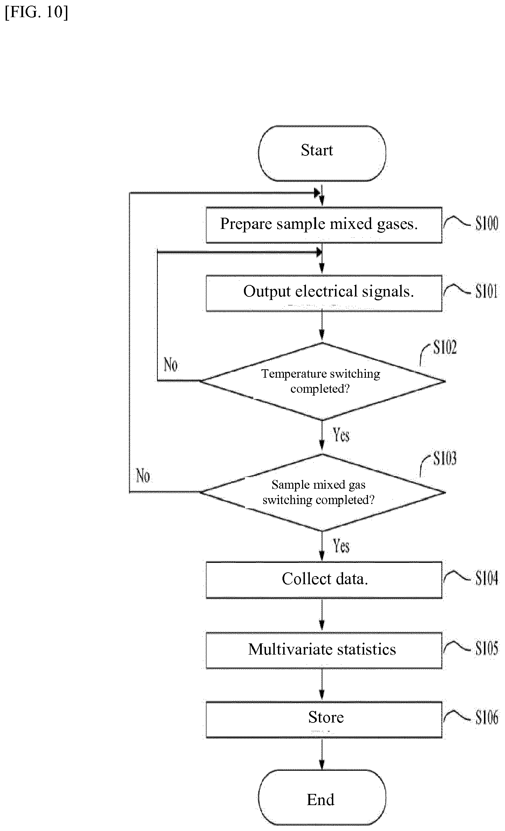

The flow chart shown in FIG. 10 is used next to explain a method for generating an equation for calculating the sum of the products of the numbers $N_H$ of hydrogen atoms that structure the molecules included in the respective mixed gases, the atomic weight of hydrogen, and the volumetric ratios of the respective molecules.

(a) In Step S100, the valve for the first flow rate controlling device 32A is opened while leaving the second through fourth flow rate controlling devices 32B through 32D, illustrated in FIG. 9, closed, to introduce the first sample mixed gas into the pipe 101 illustrated in FIG. 8. In Step S101, the measuring portion 301 measures the value of the electric signal $S_I$ from the first temperature measuring element 62 that is in contact with the first sample mixed gas, and stores it in the electric signal storage device 401. Following this, the driving circuit 203 applies a driving power $P_{H1}$ to the heating element 61 illustrated in FIG. 1 and FIG. 2, to cause the heating element 61 produce heat at 100°C. The measuring portion 301, illustrated in FIG. 8, stores, into the electric signal storage device 401, the value of the electric signal $S_{H1}$ $(T_{H1})$ from the heating element 61 that produces heat at 100°C.

(b) In Step S102, the driving circuit 303 evaluates whether or not the switching of the temperatures of the heating element 61, illustrated in FIG. 1 and FIG. 2, has been completed. If the switching to the temperature of 150°C and to the temperature of 200°C has not been completed, then processing returns to Step S101, and the driving circuit 303, illustrated in FIG. 8, causes the heating element 61, illustrated in FIG. 1 and FIG. 2, to produce heat at 150°C. The measuring portion 301, illustrated in FIG. 8, stores, into the electric signal storage device 401, the value of the electric signal $S_{H2}$ $(T_{H2})$ from the heating element 61 that is in contact with the first sample mixed gas and that produces heat at 150°C.

(c) In Step S102, whether or not the switching of the temperatures of the heating element 61, illustrated in FIG. 1 and FIG. 2, has been completed is evaluated again. If the switching to the temperature of 200°C has not been completed, then processing returns to Step S101, and the driving circuit 303, illustrated in FIG. 8, causes the heating element 61, illustrated in FIG. 1 and FIG. 2, to produce heat at 200°C. The measuring portion 301, illustrated in FIG. 8, stores, into the electric signal storage device 401, the value of the electric signal $S_{H3}$ $(T_{H3})$ from the heating element 61 that is in contact with the first sample mixed gas and that produces heat at 200°C.

(d) If the switching of the temperature of the heating element 61 has been completed, then processing advances from Step S102 to Step S103. In Step S103, an evaluation is performed as to whether or not the switching of the sample mixed gases has been completed. If the switching to the second through fourth sample mixed gases has not been completed, processing returns to Step S100. In Step S100, the valve for the first flow rate controlling device 32A is closed and the valve for the second flow rate controlling device 32B is opened while leaving the third and fourth flow rate controlling devices 32C through 32D, illustrated in FIG. 9, closed, to introduce the second sample mixed gas into the pipe 101 illustrated in FIG. 8.

(e) The loop of Step S101 through Step S102 is repeated in the same manner as for the first sample mixed gas. Moreover, the measuring portion 301 measures the value of the electric signal $S_I$ from the first temperature measuring element 62 that is in contact with the second sample mixed gas, and stores it in the electric signal storage device 401. Moreover, the measuring portion 301 stores, into the electric signal storage device 401, the values of the electric signals $S_{H1}$ $(T_{H1})$, $S_{H2}$ $(T_{H2})$, and $S_{H3}$ $(T_{H3})$ from the heating element 61 that is in contact with the second sample mixed gas and that produces heat at 100°C, 150°C, and 200°C.

(f) Thereafter, the loop of Step S100 through Step S103 is repeated. Through this, the value of the electric signal $S_I$ from the first temperature measuring element 62 that is in contact with the third sample mixed gas that is provided to the pipe 101, and the values of the electric signals $S_{H1}$ $(T_{H1})$, $S_{H2}$ $(T_{H2})$, and $S_{H3}$ $(T_{H3})$ from the heating element 61 that is in contact with the third sample mixed gas and that produces heat at 100°C, 150°C, and 200°C are stored into the electric signal storage device 401. Moreover, the value of the electric signal $S_I$ from the first temperature measuring element 62 that is in contact with the fourth sample mixed gas that is provided to the pipe 101, and the values of the electric signals $S_{H1}$ $(T_{H1})$, $S_{H2}$ $(T_{H2})$, and $S_{H3}$ $(T_{H3})$ from the heating element 61 that is in contact with the fourth sample mixed gas and that produces heat at 100°C, 150°C, and 200°C are stored into the electric signal storage device 401.

(g) In Step S104, a known value for the sum of the products of the respective numbers of hydrogen atoms $N_H$ that structure the molecules included in the first sample mixed gas, the atomic weight of hydrogen, and the volumetric ratios of the respective molecules, a known value for the sum of the products of the respective numbers of hydrogen atoms $N_H$ that structure the molecules included in the second sample mixed gas, the atomic weight of hydrogen, and the volumetric ratios of the respective molecules, a known value for the sum of the products of the respective numbers of hydrogen atoms $N_H$ that structure the molecules included in the third sample mixed gas, the atomic weight of hydrogen, and the volumetric ratios of the respective molecules, and a known value for the sum of the products of the respective numbers of hydrogen atoms $N_H$ that structure the molecules included in the fourth sample mixed gas, the atomic weight of hydrogen, and the volumetric ratios of the respective molecules, the plurality of measured values for the electric signals $S_I$ from the first temperature measuring element 62, and the plurality of measured values for the electric signals $S_{H1}$ $(T_{H1})$, $S_{H2}$ $(T_{H2})$, and $S_{H3}$ $(T_{H3})$ from the heating element 61 are inputted from the inputting device 312 into the equation generating portion 302. Moreover, the equation generating portion 302 reads out, from the electric signal storage device 401, the plurality of measured values for the electric signal $S_I$ from the first temperature measuring element 62, and the plurality of measured values for the electric signals $S_{H1}(T_{H1})$, $S_{H2}(T_{H2})$, and $S_{H3}(T_{H3})$ from the heating element 61.

(h) In Step S105, the equation generating portion 302 performs multiple linear regression analysis based on the known values for the sums of products, the plurality of measured values for the electric signals $S_I$ from the first temperature measuring element 62, and the plurality of measured values for the electric signals $S_{H1}$ $(T_{H1})$, $S_{H2}$ $(T_{H2})$, and $S_{H3}$ $(T_{H3})$ from the heating element 61. Through this multivariate statistics, the equation generating portion 302 calculates an equation with the electric signal $S_I$ from the first temperature measuring element 62, and the electric signals $S_{H1}(T_{H1})$, $S_{H2}(T_{H2})$, and $S_{H3}(T_{H3})$ from the heating element 61 as the independent variables and the sum of the products of the respective numbers of hydrogen atoms $N_H$ that structure the molecules included in the gas, the atomic weight of hydrogen, and the volumetric ratios of the respective molecules as the dependent variable. Thereafter, in Step S106, the equation generating portion 302 stores, into the equation storage device 402, the equation that has been generated, to complete the method for generating an equation as set forth in the example.

As described above, the example according to the present disclosure makes it possible to generate an equation able to calculate uniquely the sum of the products of the numbers $N_H$ of hydrogen atoms that structure the molecules included in the respective mixed gases, the atomic weight of hydrogen, and the volumetric ratios of the respective molecules.

The functioning of the gas measuring system according to the example, illustrated in FIG. 8 when measuring the sum of the products of the numbers $N_H$ of hydrogen atoms that structure the molecules included in a mixed gas to be measured, the atomic weight of hydrogen, and the volumetric ratios of the respective molecules, is explained next. For example, a mixed gas to be measured, such as a natural gas or a utility gas that includes, at unknown volume fractions, methane ($CH_4$), propane ($C_3H_8$), nitrogen ($N_2$), and carbon dioxide gas ($CO_2$) is introduced into the pipe 101. The first temperature measuring element 62 of the microchip 8 illustrated in FIG. 1 and FIG. 2 outputs an electric signal $S_I$ that is dependent on the temperature of the gas that is measured. Following this, the heating element 61 applies driving powers $P_{H1}$, $P_{H2}$, and $P_{H3}$ from the driving circuit 303 illustrated in FIG. 8. When the driving powers $P_{H1}$, $P_{H2}$, and $P_{H3}$ are applied, the heating element 61 that is in contact with the mixed gas being measured produces heat at a temperature $T_{H1}$ of 100°C, a temperature $T_{H2}$ of 150°C, and a temperature $T_{H3}$ of 200°C, for example, to output an electric signal $S_{H1}$ $(T_{H1})$ at the heat producing temperature $T_{H1}$, an electric signal $S_{H2}$ $(T_{H2})$ at the heat producing temperature $T_{H2}$, and an electric signal $S_{H3}$ $(T_{H3})$ at the heat producing temperature $T_{H3}$.

The measuring portion 301, illustrated in FIG. 8, measures the values of the electric signal $S_I$, from the first temperature measuring element 62, which is dependent on the temperature $T_I$ of the mixed gas to be measured, which is in contact with the mixed gas to be measured, which is provided to the pipe 101, and of the electric signal $S_{H1}(T_{H1})$ at the heat producing temperature $T_{H1}$, the electric signal $S_{H2}(T_{H2})$ at the heat producing temperature $T_{H2}$, and the electric signal $S_{H3}(T_{H3})$ at the heat producing temperature $T_{H3}$, from the heating element 61 that is in contact with the mixed gas to be measured, and stores the measured values into the electric signal storage device 401.

As described above, the equation storage device 402 stores an equation that has, as independent variables, the electric signal $S_I$ from the first temperature measuring element 62, the electric signal $S_{H1}(T_{H1})$ from the heating element 61 with a heat producing temperature $T_{H1}$ of 100°C, the electric signal $S_{H2}(T_{H2})$ from the heating element 61 with a heat producing temperature $T_{H2}$ of 150°C, and the electric signal $S_{H3}(T_{H3})$ from the heating element 61 with a heat producing temperature $T_{H3}$ of 200°C, and that has, as the dependent variable, the sum of the products of the respective numbers $N_H$ of hydrogen atoms that structure the molecules included in the gas, the atomic weight of hydrogen, and the volumetric ratios of the respective molecules.

The gas measuring system 20 according to the example further comprises a product summing portion 305. The product summing portion 305 calculates the sum of the products of the respective numbers of hydrogen atoms $N_H$ that structure the molecules included in the gas, the atomic weight of hydrogen, and the volumetric ratios of the respective molecules by substituting, into the independent variable that is the electric signal $S_I$ from the first temperature measuring element 62, and the independent variables that are the electric signals $S_{H1}(T_{H1})$, $S_{H2}(T_{H2})$, and $S_{H3}(T_{H3})$ from the heating element 61, the respective measured values for the electric signal $S_I$ from the first temperature measuring element 62,

and the electric signals $S_{H1}(T_{H1})$, $S_{H2}(T_{H2})$, and $S_{H3}(T_{H3})$ from the heating element 61. A product storage device 403 is also connected to the CPU 300. The product storage device 403 stores the value for sum of products calculated by the product summing portion 305.

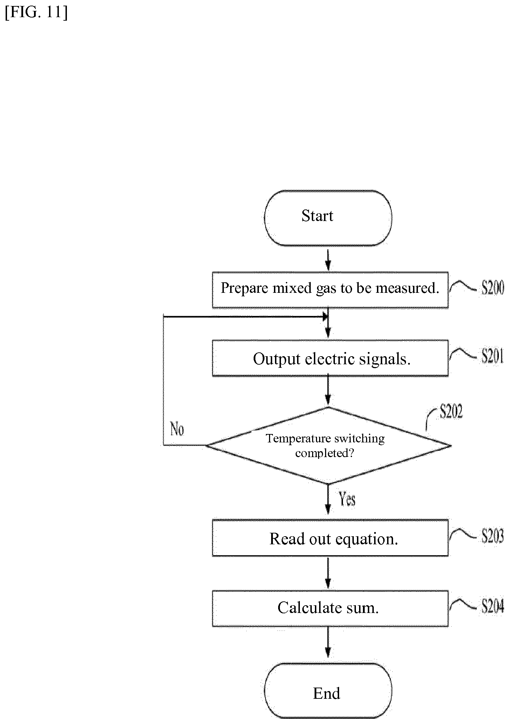

The flowchart in FIG. 11 is used next to explain the gas measuring method as set forth in the example.

(a) In Step S200, the mixed gas to be measured is introduced into the pipe 101 illustrated in FIG. 8. In Step S201, the measuring portion 301 measures the value of the electric signal $S_I$ from the first temperature measuring element 62 that is in contact with the first sample mixed gas, and stores it in the electric signal storage device 401. Following this, the driving circuit 203 applies a driving power $P_{H1}$ to the heating element 61 illustrated in FIG. 1 and FIG. 2, to cause the heating element 61 produce heat at 100°C. The measuring portion 301, illustrated in FIG. 8, stores, into the electric signal storage device 401, the value of the electric signal $S_{H1}$ ($T_{H1}$) from the heating element 61 that is in contact with the mixed gas to be measured and that produces heat at 100°C.

(b) In Step S202, the driving circuit 303, illustrated in FIG. 8, evaluates whether or not the switching of the temperatures of the heating element 61, illustrated in FIG. 1 and FIG. 2, has been completed. If the switching to the temperature of 150°C and to the temperature of 200°C has not been completed, then processing returns to Step S201, and the driving circuit 303 applies a driving power $P_{H2}$ to the heating element 61, illustrated in FIG. 1 and FIG. 2, to cause the heating element 61 to produce heat at 150°C. The measuring portion 301, illustrated in FIG. 8, stores, into the electric signal storage device 401, the value of the electric signal $S_{H2}$ ($T_{H2}$) from the heating element 61 that is in contact with the mixed gas to be measured and that produces heat at 150°C.

(c) In Step S202, whether or not the switching of the temperatures of the heating element 61, illustrated in FIG. 1 and FIG. 2, has been completed is evaluated again. If the switching to the temperature of 200°C has not been completed, then processing returns to Step S201, and the driving circuit 303 applies a driving power $P_{H3}$ to the heating element 61, illustrated in FIG. 1 and FIG. 2, to cause the heating element 61 to produce heat at 200°C. The measuring portion 301, illustrated in FIG. 8, stores, into the electric signal storage device 401, the value of the electric signal $S_{H3}$ ($T_{H3}$) from the heating element 61 that is in contact with the mixed gas to be measured and that produces heat at 200°C.

(d) If the switching of the temperature of the heating element 61 has been completed, then processing advances from Step S202 to Step S203. In Step S203, the product summing portion 305 illustrated in FIG. 8 reads out, from the equation storage device 402, an equation with the electric signal $S_I$ from the first temperature measuring element 62, and the electric signals $S_{H1}(T_{H1})$, $S_{H2}(T_{H2})$, and $S_{H3}(T_{H3})$ from the heating element 61 as the independent variables and the sum of the products of the respective numbers of hydrogen atoms $N_H$ that structure the molecules included in the gas, the atomic weight of hydrogen, and the volumetric ratios of the respective molecules as the dependent variable. Moreover, the product summing portion 305 reads out, from the electric signal storage device 401, a measured value for the electric signal $S_I$ from the first temperature measuring element 62 that is in contact with the mixed gas to be measured, and measured values for the electric signals $S_{H1}(T_{H1})$, $S_{H2}(T_{H2})$, and $S_{H3}(T_{H3})$ from the heating element 61 that is in contact with the mixed gas to be measured.

(e) In Step S204, the product summing portion 305 calculates the sum of the products of the respective numbers of hydrogen atoms $N_H$ that structure the molecules included in the mixed gas being measured, the atomic weight of hydrogen, and the volumetric ratios of the respective molecules as the dependent variable by substituting the respective measured values into the independent variable that is the electric signal $S_I$ from the first temperature measuring element 62, and the independent variables that are the electric signals $S_{H1}(T_{H1})$, $S_{H2}(T_{H2})$, and $S_{H3}(T_{H3})$ from the heating element 61. Thereafter, the product summing portion 305 stores, into the product storage device 403, the value calculated for the sum of the products, to complete the method for measuring the gas as set forth in the example.

The example according to the present disclosure, as explained above, makes it possible to measure the sum of the products of the respective numbers of hydrogen atoms $N_H$ that structure the molecules included in the mixed gas to be measured, the atomic weight of hydrogen, and the volumetric ratios of the respective molecules, from the electric signal $S_I$ from the first temperature measuring element 62, which is in contact with the mixed gas to be measured, and the electric signals $S_{H1}(T_{H1})$, $S_{H2}(T_{H2})$, and $S_{H3}(T_{H3})$ from the heating element 61, which is in contact with the mixed gas to be measured.

(Another Example)

First, 40 different sample mixed gases with known compositions were prepared. The 40 different sample mixed gases each included, as gas components, methane, propane, butane, pentane, hexane, nitrogen, and/or carbon dioxide. Following this, each of the 40 different sample mixed gases were used to obtain a plurality of measured values for the electric signal $S_I$ from the first temperature measuring element 62, illustrated in FIG. 1, and a plurality of measured values for the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61.

Thereafter, an equation was generated, based the known values for the sums of the products of the respective numbers of hydrogen atoms that structure the molecules included in each of the 40 types of sample mixed gases, the atomic weight of hydrogen, and the volumetric ratios of the respective molecules, the plurality of measured values for the electric signals $S_I$ from the first temperature measuring element 62, and the plurality of measured values for the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61, having an electric signal $S_I$ from the first temperature measuring element 62 and the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61 as independent variables, and having the sum of the products of the respective numbers of hydrogen atoms that structure the molecules included in a gas, the atomic weight of hydrogen, and the volumetric ratios of the respective molecules as the dependent variable.

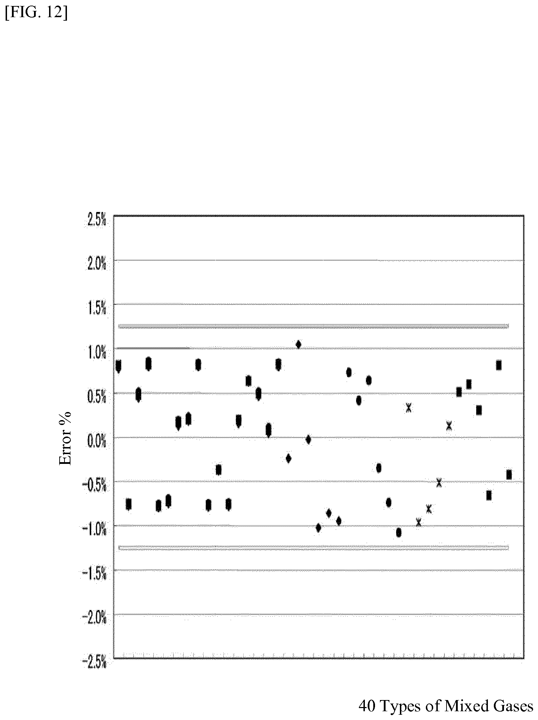

When generating the equation, as a rule it is possible to determine the equation appropriately using between 3 and 5 calibration points. When the generated equation was used to calculate the respective sums of the products of the respective numbers of hydrogen atoms that structure the molecules included in the gases, the atomic weight of hydrogen, and the volumetric ratios of the respective molecules for each of the 40 different sample mixed gases, and these were compared to the true values, the error was within a range of ± 1.25%, as illustrated in FIG. 12.

(Yet Another Example)

40 different sample mixed gases with known compositions were prepared in the same manner as in the another example. Following this, each of the 40 different sample mixed gases were used to obtain a plurality of measured values for the electric signal $S_I$ from the first temperature measuring element 62, illustrated in FIG. 1, and a plurality of measured values for the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61.

Thereafter the respective sums of the products of the respective numbers of hydrogen atoms that structure the molecules included in the gases, the atomic weight of hydrogen, and the volumetric ratios of the respective molecules for each of the 40 different sample mixed gases were calculated as shown in Equation (32) and Equation (33), above, treating ethane and butane each as mixtures of methane and propane. For example, as one of the sample mixed gases, methane was included at 90 VOL%, ethane was included at 5 VOL%, propane was included at 1 VOL%, nitrogen was included at 1 VOL%, and carbon dioxide gas was included at 2 VOL%. In this case, when the ethane and butane were decomposed into methane and propane, the volume of the methane was seen as 92 VOL%, which is the sum of the original 90 VOL% for the methane, plus 0.5 x 5 VOL% methane decomposed from the ethane and -0.5 x 1 VOL% methane decomposed from the butane. Moreover, the volume of the propane was seen as 5 VOL%, which is the sum of 0.5 x 5 VOL% propane decomposed from the ethane plus the original 1 VOL% for the propane and 1.5 x 1 VOL% propane decomposed from the butane.

Moreover, an equation was generated, based the calculated values for the sums of the products, the plurality of measured values for the electric signals $S_I$ from the first temperature measuring element 62, and the plurality of measured values for the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61, having an electric signal $S_I$ from the first temperature measuring element 62 and the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61 as independent variables, and having the sum of the products of the respective numbers of hydrogen atoms that structure the molecules included in a gas, the atomic weight of hydrogen, and the volumetric ratios of the respective molecules as the dependent variable.

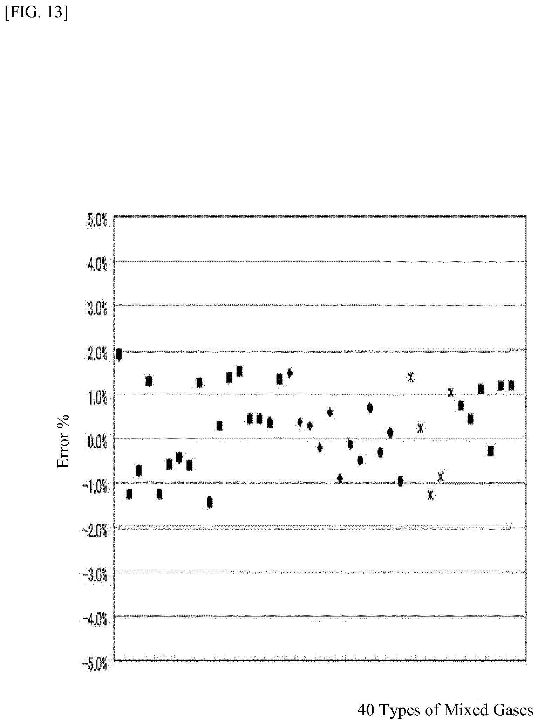

When generating the equation, as a rule it is possible to determine the equation appropriately using between 3 and 5 calibration points. When the generated equation was used to calculate the respective sums of the products of the respective numbers of hydrogen atoms that structure the molecules included in the gases, the atomic weight of hydrogen, and the volumetric ratios of the respective molecules for each of the 40 different sample mixed gases, and these were compared to the true values, the error was within a range of ± 2.0%, as illustrated in FIG. 13.

(Further Example)

A mixed gas is used as the ambient gas for the microchip 8 illustrated in FIG. 1 through FIG. 4, where the mixed gas is assumed to comprise four gas components: gas A, gas B, gas C, and gas D. When the per-unit-volume calorific value due to the carbon atoms included in the gas A is defined as $K_{AC}$, the per-unit-volume calorific value due to the carbon atoms included in the gas B is defined as $K_{BC}$, the per-unit-volume calorific value due to the carbon atoms included in the gas C is defined as $K_{CC}$, and the per-unit-volume calorific value due to the carbon atoms included in the gas D is defined as $K_{DC}$, then the per-unit-volume calorific value $Q_C$ due to the carbon atoms included in the mixed gas is obtained by summing the products of the volume fractions of the each of the individual gas components multiplied by the per-unit-volume calorific values due to the carbon atoms included in the individual gas components. Consequently, the per-unit-volume calorific value $Q_C$ due to the carbon atoms that structure the molecules included in the mixed gas is given by Equation (36), below.

$$Q_C = K_{AC} \times V_A + K_{BC} \times V_B + K_{CC} \times V_C + K_{DC} \times V_D \dots (36)$$

According to Equation (36) and Equation (18) through Equation (21), above, the per-unit-volume calorific value $Q_C$ due to the carbon atoms that structure the molecules included in the mixed gas is given by Equation (37), below, where $g_3$ is a code representing a function.

$$Q_C = g_3[M_{I1}(T_{H1}), M_{I2}(T_{H2}), M_{I3}(T_{H3})] \dots (37)$$

Moreover, has described above, the dissipating factor $M_I$ of a mixed gas depends on the electric signals $S_{H1}(T_{H1})$, $S_{H2}(T_{H2})$, and $S_{H3}(T_{H3})$ from the heating element 61 when the heat producing temperatures of the heating element 61 are $T_{H1}$, $T_{H2}$, and $T_{H3}$, and the electric signal $S_I$ of the first temperature measuring element 62 that is in contact with the mixed gas. Consequently, the per-unit-volume calorific value $Q_C$ due to the carbon atoms that structure the molecules included in the mixed gas is given by Equation (38), below, where $h_{13}$ is a code representing a function.

$$Q_C = h_{13}[S_{H1}(T_{H1}), S_{H2}(T_{H2}), S_{H3}(T_{H3}), S_I] \dots (38)$$

Furthermore, the per-unit-volume calorific value $Q_C$ due to the carbon atoms that structure the molecules included the mixed gas is correlated to the sum of the products of the respective numbers $N_C$ of carbon atoms that structure the molecules included in the mixed gas, the atomic weight of carbon (for example, 12.0107), and the volumetric ratios of the respective molecules. Consequently, based on Equation (38), above, the sum $G_C$ of the products of the respective numbers $N_C$ of carbon atoms that structure the molecules included in the mixed gas, the atomic weight of carbon, and the volumetric ratios of the respective molecules is given by Equation (39), below, where $h_{14}$ is a code representing a function.

$$G_C = h_{14}[S_{H1}(T_{H1}), S_{H2}(T_{H2}), S_{H3}(T_{H3}), S_I] \dots (39)$$

Moreover, the sum $G_C$ of the products of the respective numbers $N_C$ of carbon atoms that structure the molecules included in the mixed gas that comprises n types of gas components, the atomic weight of carbon, and the volumetric ratios of the respective molecules is given by Equation (40), below, where $h_{15}$ is a code representing a function.

$$G_C = h_{15}[S_{H1}(T_{H1}), S_{H2}(T_{H2}), S_{H3}(T_{H3}), \dots, S_{Hn-1}(T_{Hn-1}), S_I] \dots (40)$$

For example, let us assume that methane ($CH_4$) is included at 90 VOL%, ethane ($C_2H_6$) is included at 5 VOL%, propane ($C_4H_{10}$) is included at 1 VOL%, nitrogen ($N_2$) is included at 1 VOL%, and carbon dioxide gas ($CO_2$) is included at 2 VOL%. In this case, because the number of carbon atoms that structure the methane is 1, the product of the number of carbon atoms that structure the methane, the atomic weight of carbon, and the volumetric ratio of the methane is 1 x 12.0107 x 0.9 = 10.80963. Moreover, because the number of carbon atoms that structure the ethane is 2, the product of the number of carbon atoms that structure the ethane, the atomic weight of carbon, and the volumetric ratio of the ethane is 2 x 12.0107 x 0.05 = 1.20107. Moreover, because the number of carbon atoms that structure the propane is 3, the product of the number of carbon atoms that structure the propane, the atomic weight of carbon, and the volumetric ratio of the propane is 3 x 12.0107 x 0.01 = 0.360321. Moreover, because the number of carbon atoms that structure the butane is 4, the product of the number of carbon atoms that structure the butane, the atomic weight of carbon, and the volumetric ratio of the butane is 4 x 12.0107 x 0.01 = 0.480428. The number of carbon atoms that structure the nitrogen is zero. In this case, because the number of carbon atoms that structure the carbon dioxide is 1, the product of the number of carbon atoms that structure the carbon dioxide, the atomic weight of carbon, and the volumetric ratio of the carbon dioxide is 1 x 12.0107 x 0.02 = 0.240214.

Consequently, the sum of the product of the number of carbon atoms that structure the methane, the atomic weight of carbon, and the volumetric ratio of the methane, the product of the number of carbon atoms that structure the ethane, the atomic weight of carbon, and the volumetric ratio of the ethane, the product of the number of carbon atoms that structure the propane, the atomic weight of carbon, and the volumetric ratio of the propane, the product of the number of carbon atoms that structure the butane, the atomic weight of carbon, and the volumetric ratio of the butane and the product of the number of carbon atoms that structure the carbon dioxide, the atomic weight of carbon, and the volumetric ratio of the carbon dioxide is 10.80963+1.20107+0.360321+0.480428+0.240214=13.091663.

Note that, as indicated in Equations (32) through (35), above, the calculation may be performed using Equation (39) by viewing ethane ($C_2H_6$), butane ($C_4H_{10}$), pentane ($C_5H_{12}$), and hexane ($C_6H_{14}$) as a mixture of methane ($CH_4$) and propane ($C_3H_8$), with each multiplied by the respective specific factors.

In the further example, the equation generating portion 302 collects, for example, a known value for the sum of the products of the respective numbers of carbon atoms $N_C$ that structure the molecules included in a first sample mixed gas, the atomic weight of carbon, and the volumetric ratios of the respective molecules, a known value for the sum of the products of the respective numbers of carbon atoms $N_C$ that structure the molecules included in a second sample mixed gas, the atomic weight of carbon, and the volumetric ratios of the respective molecules, a known value for the sum of the products of the respective numbers of carbon atoms $N_C$ that structure the molecules included in a third sample mixed gas, the atomic weight of carbon, and the volumetric ratios of the respective molecules, and a known value for the sum of the products of the respective numbers of carbon atoms $N_C$ that structure the molecules included in a fourth sample mixed gas, the atomic weight of carbon, and the volumetric ratios of the respective molecules, the plurality of measured values for the electric signals $S_I$ from the first temperature measuring element 62, and the plurality of measured values for the electric signals $S_{H1}$ ($T_{H1}$), $S_{H2}$ ($T_{H2}$), and $S_{H3}$ ($T_{H3}$) from the heating element 61. Moreover, the equation generating portion 302 calculates an equation, through multivariate statistics, based on the collected values for the sums of the products, the values of the electric signals $S_I$, and the values of the electric signals $S_H$, with the electric signal $S_I$ from the first temperature measuring element 62, and the electric signals $S_{H1}(T_{H1})$, $S_{H2}(T_{H2})$, and $S_{H3}(T_{H3})$ from the heating element 61 as the independent variables and the sum of the products of the respective numbers of carbon atoms $N_C$ that structure the molecules included in the gas, the atomic weight of carbon, and the volumetric ratios of the respective molecules as the dependent variable. The equation storage device 402 stores the equation generated by the equation generating portion 302.

Moreover, in the further example, a product summing portion 305 calculates the sum of the products of the respective numbers of carbon atoms $N_C$ that structure the molecules included in the gas, the atomic weight of carbon, and the volumetric ratios of the respective molecules in the mixed gas by substituting, into the independent variable that is the electric signal $S_I$ from the first temperature measuring element 62, and the independent variables that are the electric signals $S_{H1}(T_{H1})$, $S_{H2}(T_{H2})$, and $S_{H3}(T_{H3})$ from the heating element 61, the respective measured values for the electric signal $S_I$ from the first temperature measuring element 62, and the electric signals $S_{H1}(T_{H1})$, $S_{H2}(T_{H2})$, and $S_{H3}(T_{H3})$ from the heating element 61. The product storage device 403 stores the value for sum of products calculated by the product summing portion 305.

The further example according to the present disclosure, as explained above, makes it possible to measure the sum of the products of the respective numbers of carbon atoms $N_C$ that structure the molecules included in the mixed gas to be measured, the atomic weight of carbon, and the volumetric ratios of the respective molecules, from the electric signal $S_I$ from the first temperature measuring element 62, which is in contact with the mixed gas to be measured, and the electric signals $S_{H1}(T_{H1})$, $S_{H2}(T_{H2})$, and $S_{H3}(T_{H3})$ from the heating element 61, which is in contact with the mixed gas to be measured.

(Yet A further Example)

40 different sample mixed gases with known compositions were prepared in the same manner as in the another example. Following this, each of the 40 different sample mixed gases were used to obtain a plurality of measured values for the electric signal $S_I$ from the first temperature measuring element 62, illustrated in FIG. 1, and a plurality of measured values for the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61.

Thereafter, an equation was generated, based the known values for the sums of the products of the respective numbers of carbon atoms $N_C$ that structure the molecules included in each of the 40 types of sample mixed gases, the atomic weight of carbon, and the volumetric ratios of the respective molecules, the plurality of measured values for the electric signals $S_I$ from the first temperature measuring element 62, and the plurality of measured values for the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61, having an electric signal $S_I$ from the first temperature measuring element 62 and the electric signals $S_{H1}(T_{H1})$, $S_{H2}(T_{H2})$, $S_{H3}(T_{H3})$, $S_{H4}(T_{H4})$, and $S_{H5}(T_{H5})$ from the heating element 61 as independent variables, and having the sum of the products of the respective numbers of carbon atoms that structure the molecules included in a gas, the atomic weight of carbon, and the volumetric ratios of the respective molecules as the dependent variable.

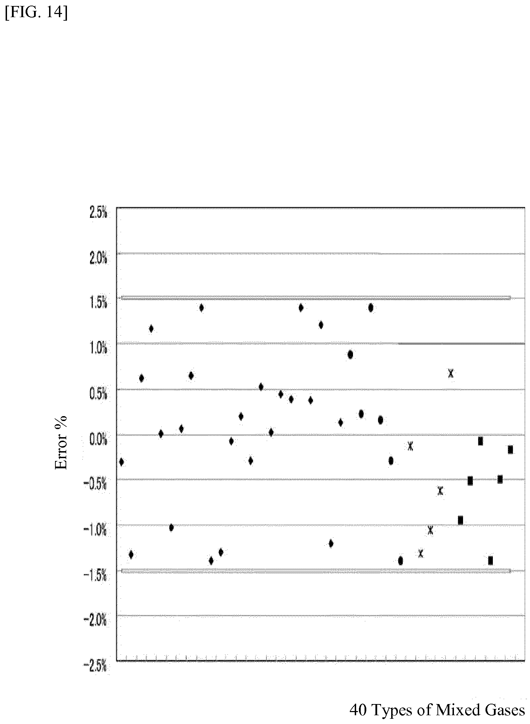

When generating the equation, as a rule it is possible to determine the equation appropriately using between 3 and 5 calibration points. When the generated equation was used to calculate the respective sums of the products of the respective numbers of carbon atoms that structure the molecules included in the gases, the atomic weight of carbon, and the volumetric ratios of the respective molecules for each of the 40 different sample mixed gases, and these were compared to the true values, the error was within a range of ± 1.5%, as illustrated in FIG. 14.

(Yet Still A Further Example)

A mixed gas is used as the ambient gas for the microchip 8 illustrated in FIG. 1 through FIG. 4, where the mixed gas is assumed to comprise four gas components: gas A, gas B, gas C, and gas D. When the per-unit-volume calorific value due to the carbon atoms and hydrogen atoms included in the gas A is defined as $K_{ACH}$, the per-unit-volume calorific value due to the carbon atoms and hydrogen atoms included in the gas B is defined as $K_{BCH}$, the per-unit-volume calorific value due to the carbon atoms and hydrogen atoms included in the gas C is defined as $K_{CCH}$, and the per-unit-volume calorific value due to the carbon atoms and hydrogen atoms included in the gas D is defined as $K_{DCH}$, then the per-unit-volume calorific value $Q_{CH}$ due to the carbon atoms and hydrogen atoms included in the mixed gas is obtained by summing the products of the volume fractions of the each of the individual gas components multiplied by the per-unit-volume calorific values due to the carbon atoms and hydrogen atoms included in the individual gas components. Consequently, the per-unit-volume calorific value $Q_{CH}$ due to the carbon atoms and hydrogen atoms included in the mixed gas is given by Equation (41), below.

$$Q_{CH} = K_{ACH} \times V_A + K_{BCH} \times V_B + K_{CCH} \times V_{CH} + K_{DC} \times V_D \dots (41)$$

According to Equation (41) and Equation (18) through Equation (21), above, the per-unit-volume calorific value $Q_{CH}$ due to the carbon atoms and hydrogen atoms that structure the molecules included in the mixed gas is given by Equation (42), below, where $g_4$ is a code representing a function.

$$Q_{CH} = g_4[M_{I1}(T_{H1}), M_{I2}(T_{H2}), M_{I3}(T_{H3})] \dots (42)$$

Moreover, has described above, the dissipating factor $M_I$ of a mixed gas depends on the electric signals $S_{H1}(T_{H1})$, $S_{H2}(T_{H2})$, and $S_{H3}(T_{H3})$ from the heating element 61 when the heat producing temperatures of the heating element 61 are $T_{H1}$, $T_{H2}$, and $T_{H3}$, and the electric signal $S_I$ of the first temperature measuring element 62 that is in contact with the mixed gas. Consequently, the per-unit-volume calorific value $Q_{CH}$ due to the carbon atoms and hydrogen atoms that structure the molecules included in the mixed gas is given by Equation (43), below, where $h_{16}$ is a code representing a function.

$$Q_{CH} = h_{16}[S_{H1}(T_{H1}), S_{H2}(T_{H2}), S_{H3}(T_{H3}), S_I] \dots (43)$$

Furthermore, the per-unit-volume calorific value $Q_{CH}$ due to the carbon atoms and hydrogen atoms that structure the molecules included the mixed gas is correlated to the sum of the products of the respective numbers $N_C$ of carbon atoms that structure the molecules included in the mixed gas, the atomic weight of carbon, and the volumetric ratios of the respective molecules and the sum of the products of the respective numbers $N_H$ of hydrogen atoms that structure the molecules included in the mixed gas, the atomic weight of hydrogen, and the volumetric ratios of the respective molecules. Consequently, based on Equation (43), above, the sum $G_{CH}$ of the products of the respective numbers $N_C$ of carbon atoms that structure the molecules included in the mixed gas, the atomic weight of carbon, and the volumetric ratios of the respective molecules that are included in the mixed gas, and the sum of the products of the respective numbers $N_H$ of hydrogen atoms that structure the molecules included in the mixed gas, the atomic weight of hydrogen, and the volumetric ratios of the respective molecules is given by Equation (44), below, where $h_{17}$ is a code representing a function.

$$G_{CH} = h_{17}[S_{H1}(T_{H1}), S_{H2}(T_{H2}), S_{H3}(T_{H3}), S_I] \dots (44)$$

Moreover, the sum $G_{CH}$ of the products of the respective numbers $N_C$ of carbon atoms that structure the molecules included in the mixed gas, the atomic weight of carbon, and the volumetric ratios of the respective molecules that are included in the n types of gas components and the sum of the products of the respective numbers $N_H$ of hydrogen atoms that structure the molecules included in the mixed gas, the atomic weight of hydrogen, and the volumetric ratios of the respective molecules is given by Equation (45), below, where $h_{18}$ is a code representing a function.

$$G_{CH} = h_{18}[S_{H1}(T_{H1}), S_{H2}(T_{H2}), S_{H3}(T_{H3}), \dots, S_{Hn-1}(T_{Hn-1}), S_I] \dots (45)$$

Note that, as indicated in Equations (32) through (35), above, the calculation may be performed using Equation (45) by viewing ethane ($C_2H_6$), butane ($C_4H_{10}$), pentane ($C_5H_{12}$), and hexane ($C_6H_{14}$) as a mixture of methane ($CH_4$) and propane ($C_3H_8$), with each multiplied by the respective specific factors.

In this yet still a further example, the equation generating portion 302, illustrated in FIG. 8, collects a known value for the sum of the products of the respective numbers $N_C$ of carbon atoms that structure the molecules included in a first sample mixed gas, the atomic weight of carbon, and the volumetric ratios of the respective molecules and a known value for the sum of the products of the respective numbers $N_H$ of hydrogen atoms that structure the molecules included in the mixed gas, the atomic weight of hydrogen, and the volumetric ratios of the respective molecules. Moreover, the equation generating portion 302 collects a known value for the sum of the products of the respective numbers $N_C$ of carbon atoms that structure the molecules included in a second sample mixed gas, the atomic weight of carbon, and the volumetric ratios of the respective molecules and a known value for the sum of the products of the respective numbers $N_H$ of hydrogen atoms that structure the molecules included in the mixed gas, the atomic weight of hydrogen, and the volumetric ratios of the respective molecules.

Moreover, the equation generating portion 302 collects a known value for the sum of the products of the respective numbers $N_C$ of carbon atoms that structure the molecules included in a third sample mixed gas, the atomic weight of carbon, and the volumetric ratios of the respective molecules and a known value for the sum of the products of the respective numbers $N_H$ of hydrogen atoms that structure the molecules included in the mixed gas, the atomic weight of hydrogen, and the volumetric ratios of the respective molecules. Moreover, the equation generating portion 302 collects a known value for the sum of the products of the respective numbers $N_C$ of carbon atoms that structure the molecules included in a fourth sample mixed gas, the atomic weight of carbon, and the volumetric ratios of the respective molecules and a known value for the sum of the products of the respective numbers $N_H$ of hydrogen atoms that structure the molecules included in the mixed gas, the atomic weight of hydrogen, and the volumetric ratios of the respective molecules.

Furthermore, the equation generating portion 302 collects the plurality of measured values for the electric signal $S_I$ from the first temperature measuring element 62, and the plurality of measured values for the electric signals $S_{H1}(T_{H1})$, $S_{H2}(T_{H2})$, and $S_{H3}(T_{H3})$ from the heating element 61. The equation generating portion 302 calculates an equation, through multivariate statistics, based on the collected values for the sums of the products, the values of the electric signals $S_I$, and the values of the electric signals $S_H$, with the electric signal $S_I$ from the first temperature measuring element 62, and the electric signals $S_{H1}(T_{H1})$, $S_{H2}(T_{H2})$, and $S_{H3}(T_{H3})$ from the heating element 61 as the independent variables and the sum of sum of the products of the respective numbers of carbon atoms $N_C$ that structure the molecules included in the gas, the atomic weight of carbon, and the volumetric ratios of the respective molecules added to the sum of the products of the respective numbers of hydrogen atoms $N_H$ that structure the molecules included in the gas, the atomic weight of hydrogen, and the volumetric ratios of the respective molecules as the dependent variable. The equation storage device 402 stores the equation generated by the equation generating portion 302.

Moreover, in the yet still a further example, a product summing portion 305 calculates the sum of sum of the products of the respective numbers of carbon atoms $N_C$ that structure the molecules included in the gas, the atomic weight of carbon, and the volumetric ratios of the respective molecules added to the sum of the products of the respective numbers of hydrogen atoms $N_H$ that structure the molecules included in the gas, the atomic weight of hydrogen, and the volumetric ratios of the respective molecules, by substituting, into the independent variable that is the electric signal $S_I$ from the first temperature measuring element 62, and the independent variables that are the electric signals $S_{H1}(T_{H1})$, $S_{H2}(T_{H2})$, and $S_{H3}(T_{H3})$ from the heating element 61, the respective measured values for the electric signal $S_I$ from the first temperature measuring element 62, and the electric signals $S_{H1}(T_{H1})$, $S_{H2}(T_{H2})$, and $S_{H3}(T_{H3})$ from the heating element 61. The product storage device 403 stores the value of the sum calculated by the product summing portion 305.

The yet still a further example according to the present disclosure, as explained above, makes it possible to measure the sum of sum of the products of the respective numbers of carbon atoms $N_C$ that structure the molecules included in the gas, the atomic weight of carbon, and the volumetric ratios of the respective molecules added to the sum of the products of the respective numbers of hydrogen atoms $N_H$ that structure the molecules included in the gas, the atomic weight of hydrogen, and the volumetric ratios of the respective molecules, from the electric signal $S_I$ from the first temperature measuring element 62, which is in contact with the mixed gas to be measured, and the electric signals $S_{H1}(T_{H1})$, $S_{H2}(T_{H2})$, and $S_{H3}(T_{H3})$ from the heating element 61, which is in contact with the mixed gas to be measured.

(Yet Another Further Example)

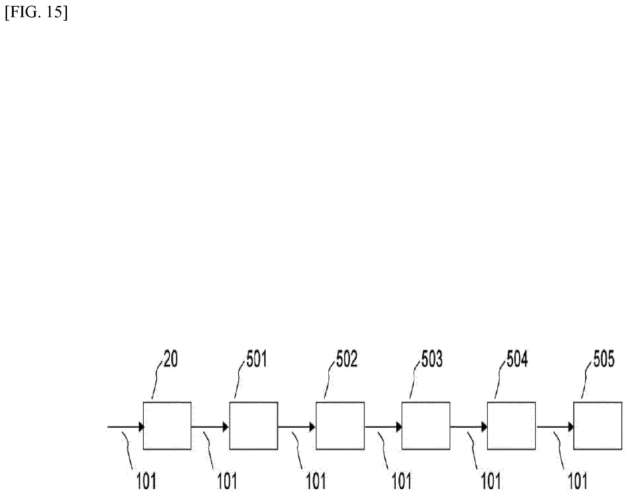

The electric power generating system as set forth in a yet another further example, as illustrated in FIG. 15, comprises: a gas measuring system 20 that is connected by a pipe 101; a flow rate controlling device 501; a reforming device 502; a shifter 503; a selective oxidizing device 504; and a fuel cell 505. The gas measuring system 20 is supplied a gas, and, as explained in the example, calculates the value of the sum of the products of the numbers of hydrogen atoms that structure the respective molecules included in the gases that flow in the pipe 101, the atomic weight of hydrogen, and the volumetric ratios of the respective molecules.

The flow rate controlling device 501 that is disposed downstream of the gas measuring system 200 controls the flow rate of the gas that flows in the pipe 101 based on the value of the sum of the products calculated by the gas measuring system 20. For example, when the sum of the products, calculated by the gas measuring system 20, is large, the flow rate of the gases that flow in the pipe 101 may be reduced because the hydrogen molecules that can be provided to the fuel cell 505 are abundant. Moreover, if the value for the total of the products, calculated by the gas measuring system 20, is small, then the flow rate of the gases that flow in the pipe 101 may be increased, because the hydrogen molecules that can be provided to the fuel cell 505 may be inadequate.

The reforming device 502 that is disposed downstream of the flow rate controlling device 501 generates the hydrogen molecules through a reforming method known as steam reformation. For example, the methane in the gas is reacted with water to produce carbon monoxide, carbon dioxide, and hydrogen. The shifter 503 that is disposed downstream from the reforming device 502 reacts the carbon monoxide that is in the gas with water to reduce the concentration of carbon monoxide in the gas through a shifting reaction that produces carbon dioxide and hydrogen molecules.

The selective oxidizing device 502 that is disposed downstream of the shifter 503 reacts the carbon monoxide that remains in the gas with oxygen in order to produce carbon dioxide, to further reduce the concentration of carbon monoxide in the gas. The fuel cell 505 that is disposed downstream from the shifter 503 is provided with a gas that is rich in hydrogen molecules and wherein the carbon monoxide concentration has been reduced, to thereby produce electricity.

The electric power generating system according to the yet another further example, set forth above, is able to predict the quantity of hydrogen molecules that are supplied to the fuel cell 505, and is able to maintain at a constant level the quantity of hydrogen molecules supplied to the fuel cell 505. Because of this, it is possible to drive the fuel cell 505 with stability. Note that the gas measuring system 20 may instead calculate the sum of the products of the numbers of carbon molecules that structure the respective molecules included in the gas, the atomic weight of carbon, and the volumetric ratios of the respective molecules.

(Other Examples)

While there are descriptions of examples as set forth above, the descriptions and drawings that form a portion of the disclosure are not to be understood to limit the present disclosure. A variety of alternate examples and operating technologies should be obvious to those skilled in the art. For example, in the example, the example explains wherein the equation storage device 402 illustrated in FIG. 8 stores an equation that has, as independent variables, electrical signals from the first temperature measuring element 62, illustrated in FIG. 1, and electrical signals from the heating element 61 at a plurality of the producing temperatures, and, as independent variables, sums of the products of the respective numbers of hydrogen atoms that structure the molecules included in the gases, the atomic weight of hydrogen, and the volumetric ratios of the respective molecules.

In contrast, as explained in Equation (24), above, the per-unit-volume calorific value $Q_H$ due to the hydrogen atoms that structure the molecules included in a mixed gas, can be obtained from an equation wherein the pressure $P_S$ of the gas and the radiation coefficients $M_{I1}(T_{H1})$, $M_{I2}(T_{H2})$, and $M_{I3}(T_{H3})$ of the gas at the respective temperatures $T_{H1}$, $T_{H2}$, and $T_{H3}$ for the heating element 61 are the variables. Moreover, the per-unit-volume calorific value $Q_H$ due to the hydrogen atoms that structure the molecules included the mixed gas is correlated to the sum of the products of the respective numbers $N_H$ of hydrogen atoms that structure the molecules included in the mixed gas, the atomic weight of hydrogen (for example, 1.00794), and the volumetric ratios of the respective molecules in the mixed gas.

Consequently, the equation storage device 402 illustrated in FIG. 8 stores a correlation between radiation coefficients and sums of products, such as an equation that has, as an independent variable, radiation coefficients of gases at a plurality of heat producing temperatures of the heating element 61, and, as independent variables, sums of the products of the respective numbers of hydrogen atoms that structure the molecules included in the gases, the atomic weight of hydrogen, and the volumetric ratios of the respective molecules. In this case, the measuring portion 301 measures the measured values for the radiation coefficients of the gas that is injected into the pipe 101, doing so with the heating element 61 producing heat at a plurality of heat producing temperatures. Note that as is explained for Equation (9), above, it is possible to measure the radiation coefficients of the gas using a microchip 8. The calculating portion 305 calculates a measured value for the sum of the products of the numbers of hydrogen atoms that structure the respective molecules included in the gas, the atomic weight of hydrogen, and the volumetric ratios of the respective molecules, by substituting, into the independent variables in the equation stored in the equation storage device 402, measured values for the radiation coefficients of the gases.

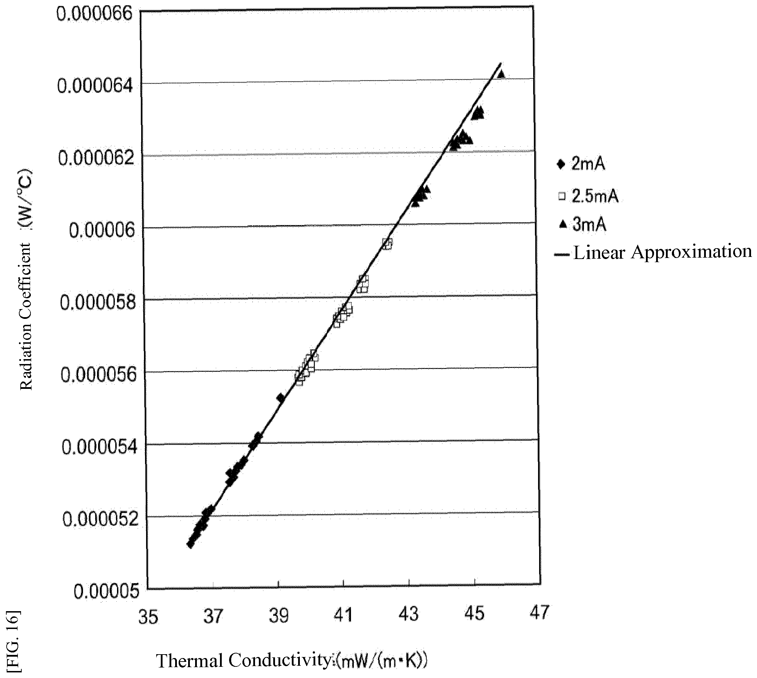

FIG. 16 illustrates the relationship between the radiation coefficient and the thermal conductivity in a mixed gas when electric currents of 2 mA, 2.5 mA, and 3 mA are produced in a heat producing resistance. As illustrated in FIG. 16, typically there is a proportional relationship between the radiation coefficient and the thermal conductivity of the mixed gas. Consequently, the equation storage device 402 illustrated in FIG. 8 stores an equation that has, as independent variables, thermal conductivities of gases at a plurality of heat producing temperatures of the heating element 61, and, as independent variables, sums of the products of the respective numbers of hydrogen atoms that structure the molecules included in the gases, the atomic weight of hydrogen, and the volumetric ratios of the respective molecules. In this case, the measuring portion 301 measures the measured values for the thermal conductivities of the gas that is injected into the pipe 101, doing so with the heating element 61 producing heat at a plurality of heat producing temperatures. The calculating portion 305 calculates a measured value for the sum of the products of the numbers of hydrogen atoms that structure the respective molecules included in the gas, the atomic weight of hydrogen, and the volumetric ratios of the respective molecules, by substituting, into the independent variables in the equation stored in the equation storage device 402, measured values for the thermal conductivities of the gases.

**Claims**

1. A gas measuring system, comprising:

   a temperature measuring element (62, 63) being in contact with a gas;
   a heating element (61) being in contact with the gas and being configured to produce heat at a plurality of heat producing temperatures;
   a measuring unit being configured to measure a value for an electric signal from the temperature measuring element (62, 63) that is dependent on the temperature of the gas, and a value for an electric signal from a heating element at each of a plurality of heat producing temperatures;
   an equation storage device (402) being configured to store an equation that includes independent variables that represent electric signals from the temperature measuring element (62, 63) and electric signals from the heating element (61) at the plurality of heat producing temperatures and a dependent variable that represents the sum of the products of the respective numbers of atoms that structure the molecules included in the gas, the atomic weight of the atoms, and the volumetric ratios of the respective molecules; and
   a calculator (305) being configured to calculate a value that represents the sum of the products of the respective numbers of atoms that structure the molecules included in the gas, the atomic weight of the atoms, and the volumetric ratios of the respective molecules, by substituting, into the independent variables in the equation, values for the electric signals from the measuring element and values for the electric signals from the heating element (61).

2. The gas measuring system as set forth in Claim 1, wherein the sum of the products of the numbers of atoms that structure each of the molecules included in the gas, the atomic weights of the atoms, and the volumetric ratios of the respective molecules is the sum of the products of the numbers of hydrogen atoms that structure each of the molecules included the gas, the atomic weight of hydrogen, and the volumetric ratios of the respective molecules.

3. The gas measuring system as set forth in Claims 1 or 2, wherein the sum of the products of the numbers of atoms that structure each of the molecules included in the gas, the atomic weights of the atoms, and the volumetric ratios of the respective molecules is the sum of the products of the numbers of carbon atoms that structure each of the molecules included the gas, the atomic weight of carbon, and the volumetric ratios of the respective molecules.

4. The gas measuring system as set forth in anyone of the preceding Claims,
   wherein the sum of the products of the numbers of atoms that structure each of the molecules included in the gas, the atomic weights of the atoms, and the volumetric ratios of the respective molecules is the sum of the sum of the products of the numbers of hydrogen atoms that structure each of the molecules included the gas, the atomic weight of hydrogen, and the volumetric ratios of the respective molecules plus sum of the products of the numbers of carbon atoms that structure each of the molecules included the gas, the atomic weight of carbon, and the volumetric ratios of the respective molecules.

5. The gas measuring system as set forth in anyone of the preceding Claims,
   wherein the mixed gas being measured is natural gas or a utility gas.

6. An electric power generating system, comprising:

   a gas measuring system according to anyone of the preceding claims, wherein the atoms are hydrogen atoms;
   a fuel cell (505) being configured to be supplied with hydrogen extracted from the gas; and
   a controlling device (501) being configured to control the amount of hydrogen provided to the fuel cell (505) based on the value indicated by the calculated product.

7. The electric power generating system as set forth in Claim 6, wherein:

the equation storage device (402) is configured to store a second equation that includes independent variables that represent electric signals from the temperature measuring element (62, 63) and electric signals from the heating element (61) at the plurality of heat producing temperatures and a dependent variable that represents the sum of the products of the respective numbers of carbon atoms that structure the molecules included in the gas, the atomic weight of carbon, and the volumetric ratios of the respective molecules; and

the calculator (305) is configured to calculate a value that represents the sum of the products of the respective numbers of carbon atoms that structure the molecules included in the gas, the atomic weight of carbon, and the volumetric ratios of the respective molecules, by substituting, into the independent variables in the first equation, values for the electric signals from the temperature measuring element (62, 63) and values for the electric signals from the heating element (61).

8. A gas measuring system according to anyone of claims 1 to 5 wherein:

the measuring unit is configured to measure a value of a gas radiation coefficient or a thermal conductivity;

the storage device (402) is configured to store a correlation between the gas radiation coefficient or a thermal conductivity, and the sum of the products of the respective numbers of atoms that structure the molecules included in the gas, the atomic weights of the atoms, and the volumetric ratios of the respective molecules; and

the calculator is configured to calculate a value representing the sum of the products of the respective numbers of atoms that structure the molecules included in the gas, the atomic weights of the atoms, and the volumetric ratios of the respective molecules based on a measured value of a gas radiation coefficient or a thermal conductivity and the correlation.

**Patentansprüche**

1. Gasmesssystem, das umfasst:

ein Temperaturmesselement (62, 63), das mit einem Gas in Kontakt steht;

ein Heizelement (61), das mit dem Gas in Kontakt steht und das so konfiguriert ist, dass es Wärme bei einer Mehrzahl von Wärmeerzeugungstemperaturen erzeugt;

eine Messeinheit, die so konfiguriert ist, dass sie einen Wert für ein elektrisches Signal vom Temperaturmesselement (62, 63), das von der Temperatur des Gases abhängig ist, und einen Wert für ein elektrisches Signal von einem Heizelement bei jeder einer Mehrzahl von Wärmeerzeugungstemperaturen misst;

eine Gleichungsspeichervorrichtung (402), die so konfiguriert ist, dass sie eine Gleichung speichert, umfassend unabhängige Variablen, die elektrische Signale vom Temperaturmesselement (62, 63) und elektrische Signale vom Heizelement (61) bei der Mehrzahl von Wärmeerzeugungstemperaturen darstellen, und eine abhängige Variable, die die Summe der Produkte der jeweiligen Zahlen von Atomen, die die Moleküle strukturieren, die im Gas enthalten sind, des Atomgewichts der Atome und der Volumenverhältnisse der jeweiligen Moleküle darstellt; und

eine Berechnungseinheit (305), die so konfiguriert ist, dass sie einen Wert berechnet, der die Summe der Produkte der jeweiligen Zahlen von Atomen, die die Moleküle strukturieren, die im Gas umfasst sind, des Atomgewichts der Atome und der Volumenverhältnisse der jeweiligen Moleküle darstellt, durch Ersetzen von Werten für die elektrischen Signale vom Messelement und Werten für die elektrischen Signale vom Heizelement (61) in den unabhängigen Variablen in der ersten Gleichung.

2. Gasmesssystem nach Anspruch 1, wobei die Summe der Produkte der Zahlen von Atomen, die jedes der Moleküle strukturieren, die im Gas umfasst sind, der Atomgewichte der Atome und der Volumenverhältnisse der jeweiligen Moleküle die Summe der Produkte der Zahlen von Wasserstoffatomen, die jedes der Moleküle strukturieren, die im Gas umfasst sind, des Atomgewichts von Wasserstoff und der Volumenverhältnisse der jeweiligen Moleküle ist.

3. Gasmesssystem nach Anspruch 1 oder 2, wobei die Summe der Produkte der Zahlen von Atomen, die jedes der Moleküle strukturieren, die im Gas umfasst sind, der Atomgewichte der Atome und der Volumenverhältnisse der jeweiligen Moleküle die Summe der Produkte der Zahlen von Kohlenstoffatomen, die jedes der Moleküle strukturieren, die im Gas umfasst sind, des Atomgewichts von Kohlenstoff und der Volumenverhältnisse der jeweiligen Moleküle ist.

4. Gasmesssystem nach einem der vorstehenden Ansprüche, wobei die Summe der Produkte der Zahlen von Atomen, die jedes der Moleküle strukturieren, die im Gas umfasst sind, der Atomgewichte der Atome und der Volumenverhältnisse der jeweiligen Moleküle die Summe der Summe der Produkte der Zahlen von Wasserstoffatomen, die jedes der Moleküle strukturieren, die im Gas umfasst sind, des Atomgewichts von Wasserstoff und der Volumenverhältnisse der jeweiligen Moleküle plus der Summe der Produkte der Zahlen von Kohlenstoffatomen, die jedes der Moleküle strukturieren, die im Gas umfasst sind, des Atomgewichts von Kohlenstoff und der Volumenverhältnisse der jeweiligen Moleküle ist.

5. Gasmesssystem nach einem der vorstehenden Ansprüche, wobei das zu messende gemischte Gas Erdgas oder ein Nutzgas ist.

6. System zum Erzeugen elektrischer Energie, das umfasst:

   ein Gasmesssystem nach einem der vorstehenden Ansprüche, wobei die Atome Wasserstoffatome sind;
   eine Brennstoffzelle (505), die so konfiguriert ist, dass sie mit aus dem Gas extrahierten Wasserstoff versorgt wird; und
   eine Steuervorrichtung (501), die so konfiguriert ist, dass sie die Menge an Wasserstoff, die der Brennstoffzelle (505) zugeführt wird, auf Basis des vom berechneten Produkt indizierten Werts steuert.

7. System zum Erzeugen elektrischer Energie nach Anspruch 6, wobei:

   die Gleichungsspeichervorrichtung (402) so konfiguriert ist, dass sie eine zweite Gleichung speichert, umfassend unabhängige Variablen, die elektrische Signale vom Temperaturmesselement (62, 63) und elektrische Signale vom Heizelement (61) bei der Mehrzahl von Wärmeerzeugungstemperaturen darstellen, und eine abhängige Variable, die die Summe der Produkte der jeweiligen Zahlen von Kohlenstoffatomen, die die Moleküle strukturieren, die im Gas enthalten sind, des Atomgewichts von Kohlenstoff und der Volumenverhältnisse der jeweiligen Moleküle darstellt; und
   die Berechnungseinheit (305) so konfiguriert ist, dass sie einen Wert berechnet, der die Summe der Produkte der jeweiligen Zahlen von Kohlenstoffatomen, die die Moleküle strukturieren, die im Gas umfasst sind, des Atomgewichts von Kohlenstoff und der Volumenverhältnisse der jeweiligen Moleküle darstellt, durch Ersetzen von Werten für die elektrischen Signale vom Temperaturmesselement (62, 63) und Werten für die elektrischen Signale vom Heizelement (61) in den unabhängigen Variablen in der ersten Gleichung.

8. Gasmesssystem nach einem der Ansprüche 1 bis 5, wobei:

   die Messeinheit so konfiguriert ist, dass sie einen Wert eines Gasstrahlungskoeffizienten oder einer Wärmeleitfähigkeit misst;
   die Speichervorrichtung (402) so konfiguriert ist, dass sie eine Korrelation zwischen dem Gasstrahlungskoeffizienten oder einer Wärmeleitfähigkeit und der Summe der Produkte der jeweiligen Zahlen von Atomen, die die Moleküle strukturieren, die im Gas umfasst sind, der Atomgewichte der Atome und der Volumenverhältnisse der jeweiligen Moleküle speichert; und
   die Berechnungseinheit so konfiguriert ist, dass sie einen Wert, der die Summe der Produkte der jeweiligen Zahlen von Atomen, die die Moleküle strukturieren, die im Gas umfasst sind, der Atomgewichte der Atome und der Volumenverhältnisse der jeweiligen Moleküle darstellt, auf Basis eines gemessenen Werts eines Gasstrahlungskoeffizienten oder einer Wärmeleitfähigkeit und der Korrelation berechnet.

**Revendications**

1. Système de mesure de gaz comprenant :

   un élément de mesure de température (62, 63) qui est en contact avec un gaz ;
   un élément chauffant (61) qui est en contact avec le gaz et qui est configuré pour produire de la chaleur à une pluralité de températures de production de chaleur ;
   une unité de mesure qui est configurée pour mesurer une valeur pour un signal électrique provenant de l'élément de mesure de température (62, 63) qui dépend de la température du gaz, et une valeur pour un signal électrique provenant d'un élément chauffant à chacune parmi une pluralité de températures de production de chaleur ; un dispositif de stockage d'équation (402) qui est configuré pour stocker une équation qui comprend des variables indépendantes qui représentent des signaux électriques provenant de l'élément de mesure de température (62, 63) et des signaux électriques provenant de l'élément chauffant (61) à la pluralité de températures de production de chaleur, et une variable dépendante qui représente la somme des produits des nombres respectifs d'atomes qui structurent les molécules incluses dans le gaz, du poids atomique des atomes, et des proportions volumétriques des molécules respectives ; et un calculateur (305) qui est configuré pour calculer une valeur qui représente la somme des produits des nombres respectifs d'atomes qui structurent les molécules incluses dans le gaz, du poids atomique des atomes, et des proportions volumétriques des molécules respectives, en substituant, dans les variables indépendantes dans l'équation, les valeurs pour les signaux électriques provenant de l'élément de mesure et les valeurs pour les signaux électriques provenant de l'élément chauffant (61).

2. Système de mesure de gaz selon la revendication 1, dans lequel la somme des produits des nombres d'atomes qui structurent chacune des molécules incluses dans le gaz, des poids atomiques des atomes, et des proportions volumétriques des molécules respectives, est la somme des produits des nombres d'atomes d'hydrogène qui structurent chacune des molécules incluses dans le gaz, du poids atomique de l'hydrogène, et des proportions volumétriques des molécules respectives.

3. Système de mesure de gaz selon la revendication 1 ou 2, dans lequel la somme des produits des nombres d'atomes qui structurent chacune des molécules incluses dans le gaz, des poids atomiques des atomes, et des proportions volumétriques des molécules respectives, est la somme des produits des nombres d'atomes de carbone qui structurent chacune des molécules incluses dans le gaz, du poids atomique du carbone, et des proportions volumétriques des molécules respectives.

4. Système de mesure de gaz selon l'une quelconque des revendications précédentes, dans lequel la somme des produits des nombres d'atomes qui structurent chacune des molécules incluses dans le gaz, des poids atomiques des atomes, et des proportions volumétriques des molécules respectives, est la somme de la somme des produits des nombres d'atomes d'hydrogène qui structurent chacune des molécules incluses dans le gaz, du poids atomique de l'hydrogène, et des proportions volumétriques des molécules respectives, plus la somme des produits des nombres d'atomes de carbone qui structurent chacune des molécules incluses dans le gaz, du poids atomique du carbone, et des proportions volumétriques des molécules respectives.

5. Système de mesure de gaz selon l'une quelconque des revendications précédentes, dans lequel le mélange gazeux qui est mesuré est du gaz naturel ou un gaz canalisé.

6. Système de génération d'énergie électrique comprenant :

   un système de mesure de gaz selon l'une quelconque des revendications précédentes, dans lequel les atomes sont des atomes d'hydrogène ;
   une pile à combustible (505) qui est configurée pour être alimentée en hydrogène extrait du gaz ; et
   un dispositif de commande (501) qui est configuré pour commander la quantité d'hydrogène fourni à la pile à combustible (505) sur la base de la valeur indiquée par le produit calculé.

7. Système de génération d'énergie électrique selon la revendication 6, dans lequel :

   le dispositif de stockage d'équation (402) est configuré pour stocker une deuxième équation qui comprend des variables indépendantes qui représentent des signaux électriques provenant de l'élément de mesure de température (62, 63) et des signaux électriques provenant de l'élément chauffant (61) à la pluralité de températures de production de chaleur, et une variable dépendante qui représente la somme des produits des nombres respectifs d'atomes de carbone qui structurent les molécules incluses dans le gaz, du poids atomique du carbone, et des proportions volumétriques des molécules respectives ; et
   le calculateur (305) est configuré pour calculer une valeur qui représente la somme des produits des nombres respectifs d'atomes de carbone qui structurent les molécules incluses dans le gaz, du poids atomique du carbone, et des proportions volumétriques des molécules respectives, en substituant, dans les variables indépendantes dans la première équation, des valeurs pour les signaux électriques provenant de l'élément de mesure de température (62, 63) et des valeurs pour les signaux électriques provenant de l'élément chauffant (61).

8. Système de mesure de gaz selon l'une quelconque des revendications 1 à 5, dans lequel :

l'unité de mesure est configurée pour mesurer une valeur d'un coefficient de rayonnement gazeux ou d'une conductivité thermique ;

le dispositif de stockage (402) est configuré pour stocker une corrélation entre le coefficient de rayonnement gazeux ou une conductivité thermique, et la somme des produits des nombres respectifs d'atomes qui structurent les molécules incluses dans le gaz, des poids atomiques des atomes, et des proportions volumétriques des molécules respectives ; et

le calculateur est configuré pour calculer une valeur représentant la somme des produits des nombres respectifs d'atomes qui structurent les molécules incluses dans le gaz, des poids atomiques des atomes, et des proportions volumétriques des molécules respectives, sur la base d'une valeur mesurée d'un coefficient de rayonnement gazeux ou d'une conductivité thermique, et de la corrélation.

[FIG. 1]

[FIG. 2]

[FIG. 3]

[FIG. 4]

[FIG. 5]

[FIG. 6]

[FIG. 7]

Temperature of the Heat Generating Resistance (°C)

[FIG. 8]

20

312 Inputting Device

313 Outputting Device

401 Electric Signal Storage Device

402 Equation Storage Device

403 Product Storage Device

300

CPU

301 Measuring Portion

302 Equation Generating Portion

305 Product Summing Portion

304 A/D Converting Circuit

303 Driving Circuit

18

8

101

[FIG. 9]

EP 2 571 086 B1

[FIG. 10]

[FIG. 11]

```
                    ┌──────────────┐
                    │    Start     │
                    └──────┬───────┘
                           │
                           ▼
        ┌──────────────────────────────────┐
        │  Prepare mixed gas to be measured.│ ~ S200
        └──────────────────┬───────────────┘
                           │
    ┌──────────────────────┤
    │                      ▼
    │      ┌──────────────────────────────┐
    │      │    Output electric signals.  │ ~ S201
    │      └──────────────┬───────────────┘
    │                     │
    │                     ▼
    │ No        ◇ Temperature switching ◇    S202
    └──────────   completed?
                           │
                          Yes
                           │
                           ▼
        ┌──────────────────────────────┐
        │     Read out equation.       │ ~ S203
        └──────────────┬───────────────┘
                       │
                       ▼
        ┌──────────────────────────────┐
        │       Calculate sum.         │ ~ S204
        └──────────────┬───────────────┘
                       │
                       ▼
                ┌──────────────┐
                │     End      │
                └──────────────┘
```

[FIG. 12]

40 Types of Mixed Gases

[FIG. 13]

40 Types of Mixed Gases

[FIG. 14]

40 Types of Mixed Gases

[FIG. 15]

[FIG. 16]

**EP 2 571 086 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011200370 A
- JP H10284104 B
- JP 2002315224 A

- US 2011185789 A1
- JP H5141999 B

**Non-patent literature cited in the description**

- A Tutorial on Support Vector Regression. Neuro-COLT Technical Report NC-TR-98-030